(19) Europäisches Patentamt
European Patent Office
Office européen des brevets

(11) Numéro de publication: **0 305 242 B1**

(12) # FASCICULE DE BREVET EUROPEEN

(45) Date de publication de fascicule du brevet: **05.01.94**

(51) Int. Cl.5: **C07J 41/00**, C07J 1/00, A61K 31/565

(21) Numéro de dépôt: **88401956.3**

(22) Date de dépôt: **28.07.88**

Le dossier contient des informations techniques présentées postérieurement au dépôt de la demande et ne figurant pas dans le présent fascicule.

(54) **Nouveaux 17-aryle stéroides, leur procédé et intermédiaires de préparation, leur application comme médicaments et les compositions pharmaceutiques les renfermant.**

(30) Priorité: **30.07.87 FR 8710794**

(43) Date de publication de la demande: **01.03.89 Bulletin 89/09**

(45) Mention de la délivrance du brevet: **05.01.94 Bulletin 94/01**

(84) Etats contractants désignés: **AT BE CH DE ES GB GR IT LI LU NL SE**

(56) Documents cités:
**EP-A- 0 057 115**
**DE-A- 2 029 569**
**FR-A- 2 268 528**
**FR-A- 2 283 905**
**FR-A- 2 380 300**
**GB-A- 2 008 119**
**US-A- 3 346 602**

**TETRAHEDRON, vol. 33, no. 6, 1977, pages 609-616, Pergamon Press, Oxford, GB; J.S. BARAN et al.: "The synthesis of 11beta-alkyl-19-norsteroids: a novel class of potent steroid hormones - I"**

(73) Titulaire: **ROUSSEL-UCLAF**
**35, Boulevard des Invalides**
**F-75007 Paris(FR)**

(72) Inventeur: **Nedelec, Lucien**
**45, Boulevard de l'Ouest**
**F-93340 Le Raincy(FR)**
Inventeur: **Nique, François**
**7, Allée Pierre Brossolette**
**F-93320 Pavillons-sous-Bois(FR)**
Inventeur: **Philibert, Daniel**
**16, rue Chevalier**
**F-94210 La Varenne Saint-Hilaire(FR)**
Inventeur: **Moguilewsky, Martine**
**37, rue Lamartine**
**F-75009 Paris(FR)**
Inventeur: **Bouton, Marie-Madeleine**
**47-49, Avenue du Dr Arnold Netter**
**F-75012 Paris(FR)**

(74) Mandataire: **Vieillefosse, Jean-Claude et al**
**Département des Brevets**
**ROUSSEL UCLAF**
**B.P no 9**
**F-93230 Romainville (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.9/3.3.3)

RECUEIL, JOURNAL OF THE ROYAL NETHERLANDS CHEMICAL SOCIETY, vol. 99, no. 10, octobre 1980, pages 311-314, Amsterdam, NL; H.J.J. LOOZEN et al.: "An approach to 11beta-isopropoxymethyl steroids"

CHEMICAL ABSTRACTS, vol. 100, no. 1, 2 janvier 1984, page 602, résumé no. 6927n, Columbus, Ohio, US; A.J. VAN DEN BROEK et al.: "Org 4333, a potent, irreversibly binding estrogen agonist", & PHARM. WEEKBL., SCI. ED., 1983, 5(4), 182-3

## Description

La présente invention concerne de nouveaux 17-aryle stéroïdes, leur procédé et des intermédiaires de préparation, leur application comme médicaments et les compositions pharmaceutiques les renfermant.

Dans la demande de brevet européen EP 0 057 115 sont décrits des produits substitués en 11$\beta$ par un radical organique comportant un azote, un phosphore ou un silicium.

Les produits de la présente demande présentent une activité antiproliférative supérieure à celle de ces produits.

Dans la demande de brevet britannique GB 2 008 119 sont décrits des produits comportant, en position 17$\alpha$, un radical aryle eventuellement substitué. Cependant, ces produits comportent, en position 11 des radicaux très différents de ceux des présents produits.

Dans les brevets américain US 3 346 602, allemand DE 2 029 569, français FR 2 283 905, 2 380 300 et 2 268 528 ainsi que dans les publications Tet. Letters, vol 33, n°6, 1977, p. 609-616; Recueil, Journ. of the Royal Netherlands Chemical Society, 99, n°10, 1980, p.311-314; Chemical Abstracts, 100, n°1, 1984, p.602, ref. 692712, sont décrits des produits stéroïdes comportant en position 17 un radical céto.

Cependant, les produits de ce type décrits dans la présente demande sont différents des produits décrits dans l'art antérieur ainsi constitué.

L'invention a pour objet les produits de formule générale I :

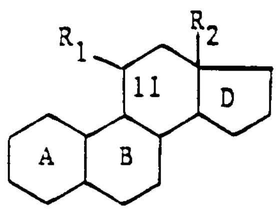

dans laquelle R1 représente

- un radical alkyle, linéaire ou ramifié, ayant de 1 à 12 atomes de carbone, ou un radical vinyle, isopropényle, allyle, 2-méthylallyle ou isobutényle, tous ces radicaux étant éventuellement substitués par :
  . les radicaux thiométhyle, thioéthyle,
  . les atomes d'halogènes,
  . le radical diméthylamino,
- un radical cycloalkyle ou cycloalkényle
- un radical phényle, benzyle ou un radical aryle hétérocyclique choisi parmi les radicaux thiényle, furyle, isothiényle, isofuryle, thiazolyle, isothiazolyle, oxazolyle, isoxazolyle, thiadiazolyle, pyridinyle ou pipéridinyle ces radicaux étant eux même éventuellement substitués par un ou plusieurs radicaux choisis parmi les radicaux :
  . alkyle ayant de 1 à 4 atomes de carbone
  . alkoxy ayant de 1 à 4 atomes de carbone
  . vinyloxy ou allyloxy
  . les atomes d'halogènes
  . trifluorométhyle,
  . alkylthio ayant de 1 à 4 atomes de carbone éventuellement oxydé sous forme de sulfoxyde ou de sulfone
  . acyle tel que acétyle, propionyle, butyryle ou benzoyle
  . un radical amino éventuellement substitué par un ou deux radicaux alkyle ayant de 1 à 8 atomes de carbone ou incorporé dans un hétérocycle comportant éventuellement un autre hétéroatome choisi dans le groupe formé par l'oxygène, l'azote et le soufre
  . un radical amino éventuellement substitué alkyle tel que amino méthyle ou aminoéthyle, diméthylamino méthyle ou diméthylamino éthyle
  . un radical amino éventuellement substitué alkyloxy tel que diméthylamino éthyloxy
  . le radical triméthylsilyle

. un radical hydroxy éventuellement acylé tel que acétoxy ou un radical de formule :

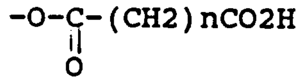

dans lequel n = 2 à 5

. un radical carboxy libre ou estérifié

. un radical cyano

. un radical aryle lui même éventuellement substitué par des radicaux alkyle, alkoxy, alkylthio, amino alkyle ou dialkyle amino tels que définis ci-dessus

étant entendu que l'atome immédiatement adjacent au carbone en 11 étant un atome de carbone, $R_2$ représente un radical méthyle ou éthyle, les cycles A et B ayant l'une des structures suivantes :

a) Soit A et B représentent le groupement :

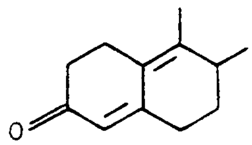

b) Soit A et B représentent le groupement :

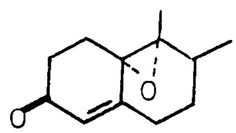

c) soit A et B représentent le groupement :

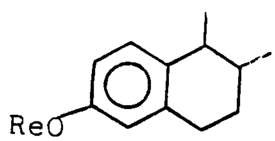

dans lequel Re représente un atome d'hydrogène, un radical alkyle ayant de 1 à 6 atomes de carbone éventuellement substitué ou un radical acyle, le cycle D ayant l'une des structures suivantes :

a) Soit D représente le groupement :

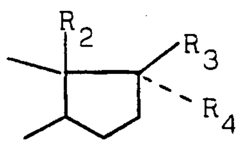

dans lequel l'un des substituants $R_3$ ou $R_4$ représente un radical hydroxyle éventuellement protégé ou acylé ou un radical alcoxyle et l'autre des substituants $R_3$ ou $R_4$ représente un radical aryle éventuellement substitué,

b) soit D représente le groupement :

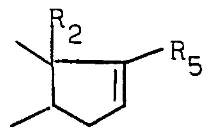

dans lequel $R_5$ représente un radical aryle éventuellement substitué, ainsi que les sels d'addition des produits de formule I avec les acides et les bases, et à l'exception du produit de formule I dans laquelle les cycles A et B représentent le groupement :

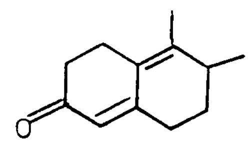

$R_1$ représente le radical :

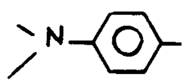

$R_2$ représente un radical méthyle, $R_3$ représente un radical hydroxyle et $R_4$ représente un radical phényle.
Lorsque D représente le groupement

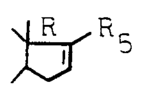

A et B représentent de préférence

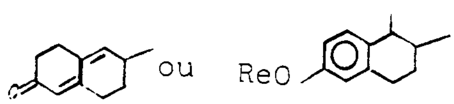

Le radical $R_1$ peut représenter un radical alkyle saturé ou insaturé, linéaire ou ramifié ayant de 1 à 12 atomes de carbone.

Il s'agit alors de préférence du radical méthyle, éthyle, propyle, isopropyle, butyle, isobutyle ou tert-butyle, n-pentyle, n-hexyle, 2-méthyl pentyle, 2,3-diméthyl butyle, n-heptyle, 2-méthylhexyle, 2,2-diméthylpentyle, 3,3-diméthyl pentyle, 3-éthylpentyle, n-octyle, 2,2-diméthylhexyle, 3,3-diméthylhexyle, 3-méthyl-3-éthylpentyle, nonyle, 2,4-diméthyl heptyle ou n-décyle. Il peut s'agir également des radicaux $vinyle_1$ isopropényle, allyle, 2-méthylallyle ou isobutényle.

Les radicaux précités peuvent être substitués. Parmi les substituants possibles, on peut citer les radicaux thioalkyle, tels que thiométhyle ou thioéthyle ; $R_1$ peut également être substitué par un ou plusieurs atomes d'halogènes tels que fluor, chlore, brome, iode ou par les radicaux amino substitués tels que diméthylamino, $R_1$ peut également représenter un radical aryle ou aralcoyle. Il s'agit alors de préférence du radical phényle ou benzyle. Ces radicaux aromatiques peuvent être substitués en ortho, méta ou para par un ou plusieurs radicaux alkyles renfermant de préférence de 1à 4 atomes de carbone ; un ou plusieurs radicaux alkoxy ayant préférentiellement de 1 à 4 atomes de carbone tels que les radicaux méthoxy, éthoxy, propyloxy, isopropyloxy, butyloxy, isobutyloxy, tert-butyloxy ; alkényloxy tel que vinyloxy ou allyloxy ; un ou plusieurs atomes d'halogène, de préférence ch lore ou fluor ; par un, ou plusieurs radicaux choisis parmi les radicaux hydroxyle, trifluorométhyle, alkylthio ayant de 1 à 4 atomes de carbone éventuellement oxydé sous forme de sulfoxyde ou de sulfone tel que méthylthio, éthylthio; acyle tel que acétyle ou propionyle, de préférence en position para; bien entendu les radicaux aryle ou aralkyle peuvent être substitués par une combinaison de ces différents radicaux tels que par exemple 3-fluoro, 4-

diméthylamino phényle.

$R_1$ peut également représenter un radical aryle hétérocyclique éventuellement substitué par les différents radicaux envisagés ci-dessus. On peut citer les radicaux thiényle, furyle, isothiényle, isofuryle, thiazolyle, isothiazolyle, oxazolyle, isoxazolyle, thiadiazolyle, pyridinyle ou pipéridinyle et les hétérocycles connus de l'homme de métier.

$R_1$ peut également représenter un radical cycloalkyle tel que cyclopropyle, cyclobutyle, cyclopentyle ou cyclohexyle,

cycloalkényle tel que cyclobutényle ou cyclopropényle.

$R_1$ représente également de préférence un radical comportant un noyau aryle substitué soit par une fonction amine éventuellement substituée par un ou deux radicaux alkyle ayant de 1 à 8 atomes de carbone, soit par un radical amino incorporé dans un hétérocycle comportant éventuellement un autre hétéroatome choisi dans le groupe formé par l'oxygène, l'azote et le soufre, tel que les radicaux morpholino ou pipéridinyle.

Le radical aryle est alors de préférence le noyau phényle.

Comme substituant sur le noyau aryle, on peut également envisager un radical amino (substitué) alkyle, tel que le radical diméthylamino méthyle, diméthylamino éthyle ; un radical amino (substitué) alkyloxy, tel que le radical diméthyl amino éthyloxy.

On peut également citer les radicaux comportant un atome de silicium tel que le radical triméthylsilyl phényle.

Les radicaux précédemment cités comportant un atome d'azote peuvent être oxydés.

De manière générale, on préfère les produits dans lesquels le substituant $R_1$ comporte un hétéroatome, de préférence l'azote ou le soufre.

Par alkyle, on entend particulièrement les radicaux méthyle, éthyle, propyle, isopropyle, butyle, sec-butyle, tert-butyle, pentyle, hexyle, 2-méthylpentyle, 2,2-diméthylpentyle.

Lorsque Re comporte un substituant, il s'agit de préférence du substituant amino ou dialkylamino tel que diméthylamino.

Par acyle, on entend en particulier acétyle, propionyle, butyryle, benzoyle.

L'expression "hydroxyle éventuellement protégé" désigne un groupement hydroxyle protégé par les groupements protecteurs classiques tels que les groupements acyle comme acétyle, chloroacétyle, trifluoroacétyle, les groupements tétrahydropyrannyle, les groupements silylés tels que triméthylsilyle, tert-butyle diméthylsilyle.

L'expression "hydroxyle éventuellement acylé" désigne un radical hydroxyle acylé par l'un des radicaux désignés ci-dessus, par exemple le radical acétyle.

Par alcoxyle, on entend plus particulièrement les radicaux dérivés des radicaux alkyles mentionnés ci -dessus et en particulier les radicaux méthoxyle, éthoxyle, propyloxy, isopropyloxy, butyloxy.

L'expression aryle comprend en général les radicaux carbocycliques ou hétérocycliques.

Parmi les radicaux à 5 chaînons, on peut citer les radicaux thiényle, furyle, thiazolyle, pyrrolyle, oxazolyle, imidazolyle, pyrazolyle, triazolyle (1,2,3 ou 1,2,4), tétrazolyle, isothiazolyle, isoxazolyle.

Parmi les radicaux à 6 chaînons, on peut citer les radicaux phényle, pyridinyle, pyridazinyle, pyrimidinyle, pyrazinyle.

L'expression "éventuellement substitué" appliquée aux radicaux aryle comprend de préférence les radicaux suivants :

- halogène : fluor, chlore, brome, iode,
- alkyle tel que méthyle, éthyle, propyle, isopropyle, butyle, isobutyle, tert-butyle
- alkoxy tel que méthoxy, éthoxy, propyloxy, isopropyloxy, butyloxy,
- alkylthio tel que méthylthio, éthylthio, propylthio, isopropylthio, butylthio,
- amino, alkylamino tel que méthylamino ou éthylamino, dialkylamino tel que diméthylamino, diéthylamino, méthyl éthylamino, chacun des radicaux dialkylamino étant éventuellement sous forme oxydée,
- amino alkyle tel que aminométhyle ou aminoéthyle,
- dialkylaminoalkyle tel que diméthylamino méthyle ou éthyle,
- dialkylaminoalkyloxy tel que diméthylamino éthyloxy,
- hydroxyle éventuellement acylé, par exemple acétoxy ou un radical de formule :

$$-O-\underset{\underset{O}{\|}}{C}-(CH_2)_nCO_2H$$

dans lequel n = 2 à 5 notamment,

- acyle tel que acétyle, propionyle, butyryle, benzoyle,
- carboxy libre, estérifié tel que alkoxy carbonyle par exemple méthoxy carbonyle ou éthoxy carbonyle,
- cyano,
- trifluorométhyle,
- aryle tel que phényle, furyle, thiényle ou aralkyle tel que benzyle, ces radicaux étant eux-mêmes éventuellement substitués par des radicaux alkyle, alkoxy, alkylthio, amino alkyle ou dialkylamino indiqués ci-dessus.

Bien entendu, l'expression "éventuellement substitué" indique qu'un ou plusieurs substituants, identiques ou différents, peuvent être présents.

Les substituants indiqués ci -dessus peuvent également être portés par le substituant $R_1$ notamment lorsque celui-ci représente un radical aryle.

Selon la valeur des substituants portés par les radicaux $R_1$, $R_3$, $R_4$ ou $R_5$ les produits de formule I peuvent former des sels avec les acides ou les bases ; c'est ainsi que si l'un au moins des radicaux $R_1$, $R_3$, $R_4$ ou $R_5$ comporte une fonction amino salifiable, les produits de formule I peuvent former des sels avec les acides. Il peut alors s'agir des sels, des acides chlorhydrique, bromhydrique, nitrique, sulfurique, phosphorique, acétique, formique, propionique, benzoïque, maléique, fumarique, succinique, tartrique, citrique, oxalique, glyoxylique, aspartique, alcane sulfonique tels que les acides méthane ou éthane sulfoniques, arylsulfonique, tels que les acides benzène ou paratoluène sulfonique et arylcarboxylique.

Par contre, si l'un au moins des radicaux $R_1$, $R_3$, $R_4$ ou $R_5$ comporte une fonction carboxy, elle peut être salifiée par un reste de base. On peut alors citer un sel de sodium, de potassium, de lithium, de calcium, de magnésium ou d'ammonium. On peut aussi citer les sels des bases organiques suivantes, la méthylamine, la propylamine, la triméthylamine, la diéthylamine, la triéthylamine, la N,N-diméthyléthanolamine, le tris (hydroxyméthyl) amino méthane, l'éthanolamine, la pyridine, la picoline, la dicyclohexylamine, la morpholine, la benzylamine, la procaïne, la lysine, l'arginine, l'histidine, la N-méthylglucamine.

La présente invention a notamment pour objet les produits de formule I telle que définie ci-dessus dans laquelle $R_1$ représente un radical aryle éventuellement substitué.

Parmi les valeurs du radical aryle qui peuvent être prises dans la liste donnée ci-dessus, on préfère le radical phényle éventuellement substitué. Parmi les substituants préférés du radical phényle, on peut citer les radicaux diméthylamino, méthylthio, méthoxy, acétyle et propionyle, les substituants sont de préférence en position para.

La présente invention a notamment pour objet les produits de formule I telle que définie ci-dessus dans laquelle A et B représentent le groupement :

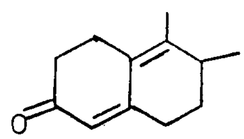

La présente invention a notamment pour objet les produits de formule I telle que définie ci-dessus dans laquelle $R_3$ représente un radical hydroxyle ou méthoxy et $R_4$ représente un radical aryle éventuellement substitué.

Parmi les valeurs du radical aryle qui peuvent êtreprises dans la liste donnée ci-dessus pour $R_4$, on préfère le radical phényle éventuellement substitué. Parmi les substituants préférés du radical phényle, on peut citer les radicaux méthylthio, méthoxy, diméthylamino, hydroxyle ou méthyle. Ces valeurs préférées de $R_4$ peuvent également convenir pour la valeur $R_5$.

L'invention a spécialement pour objet, les produits décrits ci-après dans la partie expérimentale et en particulier

- la 11béta,17alpha-bis/4-(diméthylamino) phényl/ 17béta-hydroxy estra-4,9-dièn-3-one,
- la 11béta-/4-(diméthylamino) phényl/ 17béta-hydroxy 17alpha-(3-méthoxy phényl) estra-4,9-dièn-3-one.

L'invention a également pour objet un procédé de préparation des produits de formule I telle que définie ci-dessus caractérisé en ce que :

a) soit l'on soumet un produit de formule II :

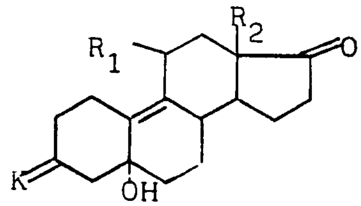

(II)

dans laquelle $R_1$ et $R_2$ ont la signification indiquée ci-dessus et K représente un groupe cétonique protégé,

i) d'abord à l'action d'un réactif organométallique dérivé d'un radical aryle éventuellement substitué que peut représenter $R_3$ ou $R_4$,

ii) puis éventuellement ou bien à une séparation des isomères obtenus ou bien à une réaction de déshydratation en position 16(17),

iii) puis éventuellement, dans un ordre quelconque, à l'action d'un agent de protection, d'alkylation ou d'acylation du radical hydroxy que représente $R_3$ ou $R_4$ et nécessairement à l'action d'un agent de déshydratation susceptible de libérer la fonction cétone, pour obtenir les produits de formules IA :

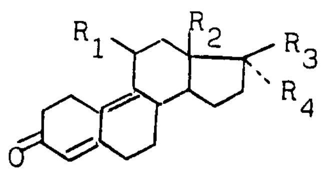

(IA)

et I'A

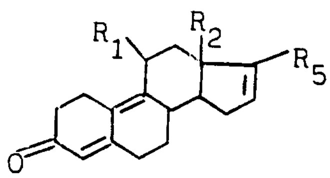

(I'A)

formules dans lesquelles $R_1$, $R_2$, $R_3$, $R_4$ et $R_5$ ont la signification indiquée ci-dessus, produit de formules IA ou I'A que, si désiré, l'on traite ou bien par un réactif d'oxydation pour obtenir respectivement le produit de formule IB :

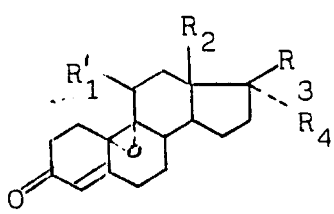

(IB)

et I'B :

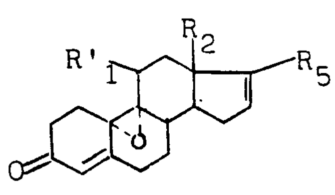

(I'B)

formules IB et I'B dans lesquelles $R_2$, $R_3$, $R_4$ et $R_5$ ont la signification indiquée ci-dessus et $R'_1$ a la signification indiquée ci-dessus pour $R_1$, étant entendu toutefois que $R'_1$ comporte un atome d'azote oxydé si $R_1$ comporte un atome d'azote, produits de formules IB et I'B dans lesquelles le radical $R'_1$ comporte un atome d'azote oxydé que si désiré l'on traite par un agent de réduction pour obtenir un produit de formule IB ou I'B dans laquelle $R'_1$ comporte un atome d'azote non oxydé,
ou bien l'on traite les produits de formules IA et I'A par un agent d'aromatisation puis éventuellement par un réactif d'alkylation ou d'acylation pour obtenir respectivement les produits de formule IC :

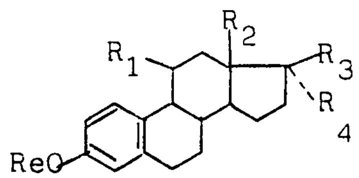

(IC)

et I'C

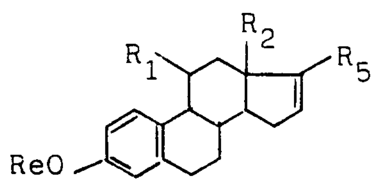

(I'C)

formules IC et I'C dans lesquelles Re, $R_1$, $R_2$, $R_3$, $R_4$ et $R_5$ ont la signification indiquée ci-dessus,
b) soit l'on soumet un produit de formule III :

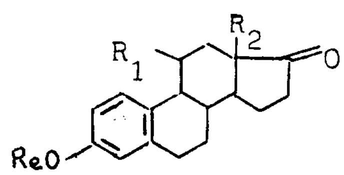

(III)

dans laquelle $R_1$ et $R_2$ ont la signification indiquée ci-dessus, à l'action d'un réactif organométalli que dérivé d'un radical aryle éventuellement substitué que peut représenter $R_3$ ou $R_4$ puis éventuellement ou bien dans un ordre quelconque à une séparation des isomères obtenus et à l'action éventuelle d'un agent de protection, d'alkylation ou d'acylation du radical hydroxy que représente $R_3$ ou $R_4$ ou bien à une réaction de déshydratation en position 16(17) et que si désiré l'on soumet les produits de formules IA, I'A, IB, I'B, IC et I'C à l'action d'une base ou d'un acide pour obtenir les sels correspondants.

Le groupement cétonique bloqué que représente C = K peut par exemple être un groupe cétal cyclique ou non cyclique, thiocétal, oxime ou méthyloxime.

L'organo métallique dérivé d'un radical aryle éventuellement substitué que peut représenter $R_3$ ou $R_4$ est de préférence choisi parmi les magnésiens de formule : ArMgHal, les lithiens de formule : ArLi, formules dans lesquelles Hal représente un atome d'halogène et Ar représente un radical aryle éventuellement substitué.

Dans un mode préféré d'exécution du procédé, Hal représente un atome de chlore, de brome ou d'iode, de préférence de brome. On opère de préférence en présence de chlorure de cérium avec lequel on fait agir le magnésien ou le lithien avant introduction des produits II ou III. le milieu réactionnel peut ensuite être soumis à un acide fort tel que l'acide chlorhydrique, l'acide nitrique ou l'acide sulfurique.

Lors de la mise en oeuvre de cette étape, on obtient généralement un mélange de produits de formules :

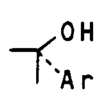

et

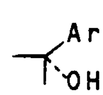

dans lesquelles Ar représente un radical aryle éventuellement substitué. On préfère obtenir de facon prépondérante ou exclusive, le produit de formule :

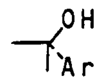

c'est-à-dire un produit de formule II' :

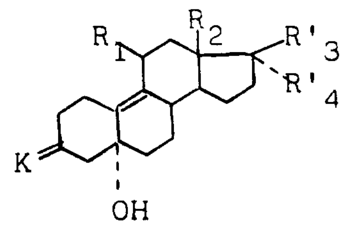

(II')

dans laquelle $R'_3$ représente un radical hydroxy et $R'_4$ représente un radical aryle éventuellement substitué. Pour obtenir ce produit, on soumet de préférence le mélange obtenu par action d'un organo métallique dérivé d'un radical aryle éventuellement substitué sur un produit de formule II à l'action des techniques usuel les de cristallisation ou de chromatographie, de préférence la chromatographie.

La réaction de déshydratation en position 16(17) a pour but de transformer les produits de formule IIa :

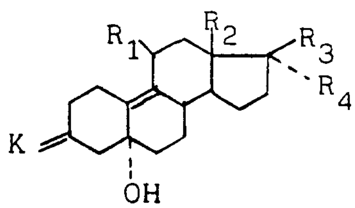

(IIa)

en produits de formule IIb :

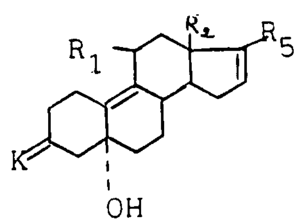

(IIb)

formules dans lesquelles K, $R_1$, $R_2$, $R_3$, $R_4$ et $R_5$ ont la signification indiquée ci-dessus.

Cette réaction peut être effectuée très facilement dans certains cas et il a été constaté par exemple que ladite réaction de déshydratation peut intervenir lors de la chromatographie tendant à séparer les deux isomères en position 17.

Dans un mode de réalisation préféré du procédé, l'agent de déshydratation capable de libérer la fonction cétone est une résine sulfonique (forme acide), par exemple une résine sulfonique du commerce à support de polystyrène ou à support de polymère styrène/divinylbenzène, mais on peut également utiliser un acide minéral tel que l'acide chlorhydrique ou l'acide sulfurique dans un alcanol inférieur ou l'acide perchlorique dans l'acide acétique ou un acide sulfonique comme l'acide paratoluène sulfonique ou l'acide acétique.

L'éventuelle protection du radical hydroxyle est effectuée selon les méthodes usuelles. On utilise un dérivé d'un groupement protecteur indiqué ci-dessus, par exemple, un halogénure tel que le chlorure de triméthylsilyle.

L'alkylation est effectuée également selon les méthodes classiques. On utilise, par exemple, un halogénure ou un sulfate d'alkyle tel que l'iodure ou le sulfate de méthyle. On préfère l'halogénure.

L'acylation éventuelle est également effectuée selon les méthodes usuelles. On utilise, par exemple, un halogénure d'acyle tel que le chlorure d'acétyle ou un anhydride mixte ou symétrique tel que l'anhydride acétique.

La déshydratation totale ou partielle en position 16(17) peut également intervenir lors de la réaction de déshydration en position 4(5) capable de libérer la fonction cétone effectuée sur les produits de formule IIa.

L'agent d'oxydation que l'on utilise pour transformer les produits de formules IA et I'A en produits de formules IB et I'B respectivement est de préférence un peracide comme l'acide métachloroperbenzoïque, l'acide peracétique ou l'acide perphtalique. Lorsque l'on désire obtenir un composé dans lequel seul l'atome d'azote du radical $R_1$ est oxydé, on utilise un équivalent d'agent d'oxydation. Lorsque l'on désire obtenir un composé dans lequel, en outre, B et C forment un pont époxyde, on utilise deux équivalents d'agent d'oxydation.

L'agent de réduction sélectif de la fonction N-oxyde est de préférence la triphénylphosphine et l'on peut opérer par exemple au sein de l'acide acétique.

L'agent d'aromatisation que l'on utilise de préférence pour transformer les produits de formules IA et I'A en produits de formules IB et I'B peut être choisi parmi les réactifs suivants :

a. l'hydroxyde de palladium déposé sur magnésie, au sein d'un alcanol inférieur tel que le méthanol ;

b. un halogénure d'acyle, par exemple le bromure d'acétyle, eventuellement en mélange avec un anhydride d'acide tel que l'anhydride acétique, au sein d'un solvant comme par exemple le chlorure de méthylène. On traite ensuite par une base comme par exemple la soude, la potasse ou le bicarbonate de potassium.

L'alkylation et l'acylation éventuel les des produits de formules IC et I'C est réalisée de facon usuelle.

On opère par exemple à l'aide d'un halogénure ou d'un sulfate d'alkyle. On utilise de préférence l'iodure d'alkyle.

La réaction d'un organométallique sur le produit de formule (III, la séparation et les réactions des isomères obtenus, d'action de protection, d'alkylation ou d'acylation du radical hydroxy effectuées sur le produit de formule I obtenu à partir des produits de formule III ansi que la déshydratation subséquente sont effectuées dans les conditions indiquées ci-dessus.

L'éventuelle formation des sels avec les acides ou les bases est effectuée dans les conditions usuel les.

Il est évident que des variantes mineures de procédé font également partie du procédé revendiqué par la présente demande.

C'est ainsi, par exemple, que les réactions éventuelles de protection, d'alkylation ou d'acylation du radical hydroxyle que peut représenter $R_3$ ou $R_4$ peuvent être effectuée sur les produits de formule IB ou IC lorsque, dans ces formules, $R_3$ ou $R_4$ représente un radical hydroxy.

L'action d'un organo magnésien sur les produits de formule III conduit directement à des produits de formules IC. A partir de ces produits, il est évident qu'on peut éventuellement effectuer, dans un ordre quelconque une séparation des isomères et/ou une réaction de protection, d'alkylation ou d'acylation du radical hydroxy que peut représenter $R_3$ ou $R_4$.

Alternativement, on peut soumettre les produits de formules IC obtenus à une réaction de déshydratation en position 16(17) pour obtenir les produits de formules IC indiquées ci-dessus.

La réaction éventuelle de salification est effectuée sur les produits de formule I quelle que soit leur mode de préparation.

Les produits de formule I ainsi que leurs sels d'addition avec les acides et les bases pharmaceutiquement acceptables sont des produits particulièrement intéressants du point de vue pharmacologique ; ils possèdent, en particulier, des propriétés anti-prolifératives.

Contrairement aux produits de formule I dans lesquels les cycles A et B représentent les groupements :

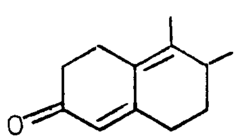

et

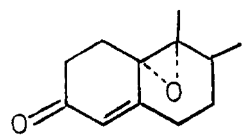

qui sont pratiquement dénués de propriétés estrogéniques et/ou anti-estrogéniques, les produits de formule I dans lesquels les cycles A et B représentent le groupement :

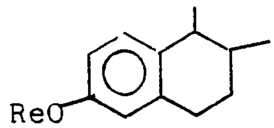

peuvent présenter de tel les propriétés estrogéniques et/ou anti-estrogéniques.

Ces propriétés rendent les produits de formule I utilisables dans le traitement des carcinomes hormonodépendants, comme, par exemple, les carcinomes mammaires et ses métastases. Ces propriétés rendent les produits de formule I ainsi que leurs sels d'addition avec les acides et les bases pharmaceutiquement acceptables également utilisables dans le traitement des tumeurs bénignes du sein.

Les propriétés estrogéniques et/ou anti-estrogéni ques que peuvent également présenter les produits de formule I dans lesquels les cyc les A et B représentent le groupement :

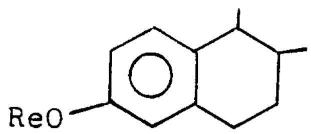

ainsi que leurs sels d'addition avec les acides et les bases pharmaceutiquement acceptables les rendent utilisables également dans le traitement des troubles liés à une hypofolliculinie, par exemple les aménorrhées, les dysménorrhées, les avortements répétés, les troubles prémenstruels ainsi que le traitement de la ménopause.

Les produits de formule I présentent également des activités progestomimétiques et principalement antiprogestomimétiques.

Ces produits de formule (I) possèdent également une activité glucocorticoïde et/ou antiglucocorticoïde comme le montrent les résultats des affinités sur récepteurs exposés ci-après.

Les produits de formule (I) ainsi que leurs sels pharmaceutiquement acceptables qui possèdent des propriétés antiprogestomimétiques peuvent être utilisés comme contraceptifs ou abortifs ; ils peuvent aussi être utilisés contre les dérèglements hormonaux.

Certains produits de formule (I) ainsi que leurs sels pharmaceutiquement acceptables, peuvent également présenter des propriétés progestomimétiques et peuvent ainsi être employés dans le traitement des aménorrhées, des dysménorrhées et des insuffisances lutéales.

Les produits de formule (I) qui possèdent des propriétés antiglucocorticoïdes ainsi que leurs sels pharmaceutiquement acceptables peuvent donc être utilisés comme médicaments pour lutter principalement contre les effets secondaires des glucocorticoïdes, ils permettent de lutter également contre les troubles dus à une hypersecrétion de glucocorticoïdes et notamment contre le vieillissement en général et plus particulièrement contre l'hypertension, l'athérosclérose, l'ostéoporose, le diabète, l'obésité ainsi que l'immunodépression et l'insomnie.

L'invention a donc pour objet, à titre de médicaments, les produits de formule I ainsi que leurs sels d'addition avec les acides et les bases pharmaceutiquement acceptables, c'est-à-dire non toxiques aux doses utilisées.

L'invention a plus particulièrement pour objet, à titre de médicaments, les produits de formule I telle que définie ci-dessus dans laquelle $R_1$ représente un radical aryle éventuellement substitué ainsi que ceux dans laquelle A et B représentent le groupement :

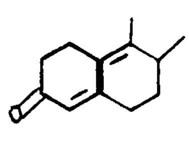

ainsi que ceux dans laquelle $R_3$ représente un radical hydroxyle ou méthoxy et $R_4$ représente un radical aryle éventuellement substitué.

L'invention a plus particulièrement pour objet, à titre de médicament, les produits de formule I décrits ci-après dans les exemples et spécialement :

- la 11béta,17alpha-bis/4-(diméthylamino) phényl/ 17béta-hydroxy estra 4,9-dien-3-one,

- la 11béta-/4-(diméthylamino) phényl/ 17béta-hydroxy 17alpha-(3-méthoxy phényl) estra-4,9-dien-3-one.

La posologie utile varie en fonction de l'affection à traiter et de la voie d'administration ; elle peut varier par exemple de 1 mg à 1 g et de préférence de 1 à 100 mg par jour chez l'adulte par voie orale.

Les nouveaux produits de formule I et leurs sels, tels que définis ci-dessus peuvent être employés pour préparer des compositions pharmaceutiques renfermant, à titre de principe actif, l'un au moins desdits produits.

Les produits de formule I et leurs sels sont utilisés par voie digestive, parentérale ou locale. Ils peuvent être prescrits sous forme de comprimés simples ou dragéifiés, de gélules, de granulés, de suppositoires, d'ovules, de préparations injectables, de pommades, de crèmes, de gels, lesquels sont préparés selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention a donc pour objet les compositions pharmaceutiques renfermant comme principe actif au moins un produit de formule I.

Les produits de formule II utilisés au départ du procédé de préparation des produits de formule I sont connus ou peuvent être préparés par des méthodes usuel les.

On trouve la description de tels produits par exemple dans le brevet européen EP 0.057.115. On trouvera également ciaprès par exemple au stade A des exemples 1 et 7 des méthodes de préparation de tels produits.

Les produits de formule III peuvent être préparés à partir par exemple des produits de formule II. On trouvera une méthode de préparation des produits de formule III aux stades A et B de l'exemple 14. De manière générale, les produits de formule III sont préparés en soumettant les produits de formule II d'abord à une réaction de déshydratation susceptible de libérer la fonction cétone dans les conditions indiquées ci-dessus. On traite ensuite les produits de formule III' ainsi obtenus :

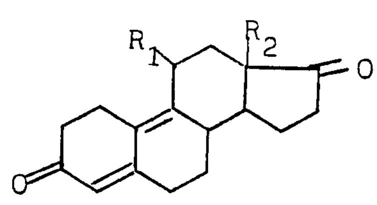

(III')

soit par un réactif d'oxydation pour obtenir un produit de formule III" :

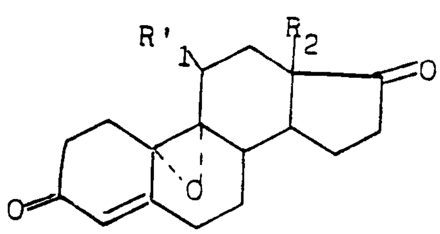

III"

dans laquelle R'$_1$ a la valeur indiquée ci-dessus puis éventuellement par un agent de réduction pour obtenir, dans les conditions indiquées ci-dessus, un produit de formule III" dans laquelle R'$_1$ représente un radical comportant un atome d'azote non oxydé,

soit par un agent d'aromatisation puis éventuellement par un réactif d'alkylation ou d'acylation pour obtenir un produit de formule III :

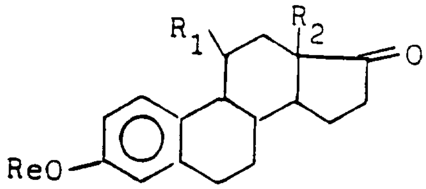

(III)

L'invention a également pour objet à titre de produits industriels nouveaux, les produits répondant aux formules suivantes :

3,3-(1,2 éthanediyl) acétal cyclique de 5 alpha-hydroxy 11 béta-(4-méthoxy phényl) estr-9-en-3,17-dione.

3,3-(1,2-éthanediyl) acétal cyclique de 5 alpha-hydroxy 11 béta-/4-(méthylthio) phényl/ estr-9-en-3,17-dione.

11 béta-/4-(diméthylamino) phényl/estra-4,9-diène 3,17-dione.

11 béta-/4-(diméthylamino) phényl/ 3-hydroxy estra 1,3,5(10)-trièn-17-one.

En plus des produits décrits dans les exemples qui illustrent l'invention sans toutefois la limiter, les produits suivants peuvent être obtenus dans le cadre de la présente invention,

1) <u>Produits de formules</u> :

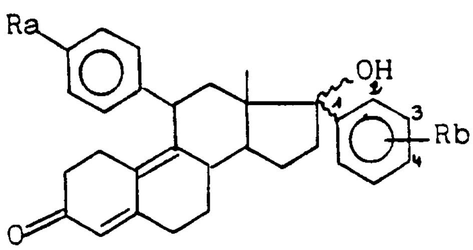

| Ra | Rb |
|---|---|
| $COCH_3$ | $4-N(CH_3)_2$ |
| $N-(CH_3)_2$ | $4-COCH_3$ |
| $SCH_3$ | $4-SCH_3$ |
| $SCH_3$ | $3SCH_3$ |
| $N-(CH_3)_2$ ↓ O | $N-(CH_3)_2$ ↓ O |
| $(CH_3)_2N-(CH_2)_2-O-$ | $3-OH$ |
| $(CH_3)_2N-(CH_2)_2-O-$ | $4-N(CH_3)_2$ |

2) <u>Produits de formules</u> :

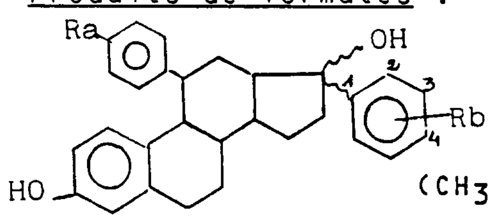

| Ra | Rb |
|---|---|
| $N-(CH_3)_2$ | $3-OH$ |
| $(CH_3)_2N-(CH_2)_2-O-$ | $3-OH$ |
| $(CH_3)_2N-(CH_2)_2-O-$ | $4-N(CH_3)_2$. |

Les traits ondulés signifient que les radicaux OH et

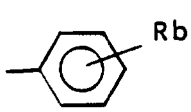

peuvent se trouver dans les 2 configurations possibles. On préfère les produits dans lesquels le substituant OH est en position béta et le substituant

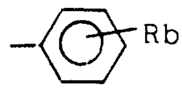

en position alpha

3) Produits de formules :

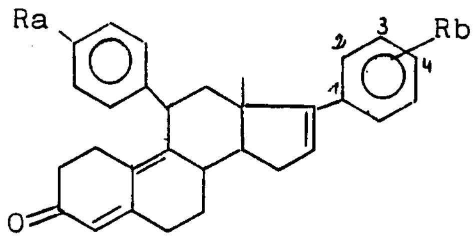

| Ra | Rb |
|---|---|
| $COCH_3$ | $4\text{-}N(CH_3)_2$ |
| $N\text{-}(CH_3)_2$ | $4\text{-}COCH_3$ |
| $SCH_3$ | $4\text{-}SCH_3$ |
| $SCH_3$ | $3\text{-}SCH_3$ |
| $N\text{-}(CH_3)_2$ ↓ O | $N\text{-}(CH_3)_2$ ↓ O |
| $(CH_3)_2\text{-}N\text{-}(CH_2)_2\text{-}O\text{-}$ | 3-OH |
| $(CH_3)_2\text{-}N\text{-}(CH_2)_2\text{-}O\text{-}$ | $4\text{-}N\text{-}(CH_3)_2$ |

4) Produits de formules :

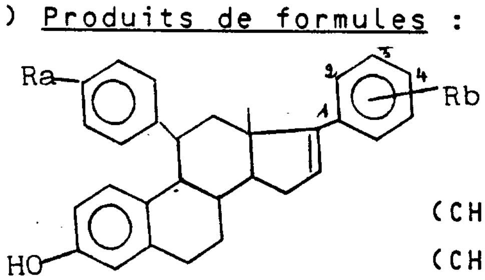

| Ra | Rb |
|---|---|
| $N\text{-}(CH_3)_2$ | 3-OH |
| $(CH_3)_2\ N\text{-}(CH_2)_2\text{-}O\text{-}$ | 3-OH |
| $(CH_3)_2\ N\text{-}(CH_2)_2\text{-}O\text{-}$ | $4\text{-}N(CH_3)_2$ |

Les exemples suivants illustrent l'invention sans la limiter.

Exemple 1 : 11béta, 17alpha-bis(4-méthoxy phényl) 17 béta-hydroxy estra-4,9-dièn-3-one.

Stade A : 3,3-(1,2 éthanediyl) acétal cyclique de 5 alpha - hydroxy 11 Béta-(4-méthoxy phényl) estr-9-en-3,17-dione.

On refroidit à -20 °C, 78 cm3 d'une solution 0,77M de bromure de (4-méthoxy phényl) magnésium dans le tétrahydrofuranne, introduit 600 mg de chorure cuivreux anhydre, agite 15 minutes et ajoute en 30 minutes à -20 °C, 6,6 g de 3,3-(1,2-éthanediyl) acétal cyclique de 5alpha, 10alpha-époxy estr9(11)èn-3,17-dione en solution dans 66 cm3 de tétrahydrofuranne anhydre. Après une heure à -20 ° C, on verse le mélange réactionnel sur 400 cm3 de solution de chlorure d'ammonium et de glace. On extrait à l'acétate d'éthyle, lave à l'eau salée, sèche et amène à sec. On dissout le résidu dans 20 cm3 d'acétate d'éthyle bouillant, refroidit à 0-5 °C, essore 5,5 g de produit que l'on dissout dans 100 cm3 de chlorure de méthylène bouillant, ajoute de l'éther isopropylique et isole 4,84 g de 3,3-(1,2-éthanediyl) acétal cyclique de 5alpha-hydroxy 11béta-(4-méthoxy phényl) estr-9-èn-3,17-dione.

On amène à sec les liqueurs-mères de cristallisation, chromatographie le résidu sur silice en éluant par un mélange cyclohexane-acétate d'éthyle (6-4) et obtient 1,9 g de produit attendu. F = 130 °C et 1,85 g du produit 17-céto obtenu ci-dessus.

Spectre IR $CHCl_3$

OH : 3510 $cm^{-1}$ de type 5-OH
C=O : 1732 $cm^{-1}$

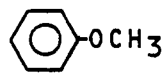 :

1608 $cm^{-1}$ - 1580 $cm^{-1}$ - 1510 $cm^{-1}$

Stade B : 11béta, 17alpha-bis(4-méthoxy phényl) 17béta-hydroxy estra-4,9-dièn-3-one.

On dissout 547 mg de produit obtenu (5alpha, 17béta-diol) au stade A dans 20 cm3 de méthanol, ajoute 0,5 cm3 d'acide chlorhydrique 2N, agite une heure à température ambiante.

On ajoute 100 cm3 d'eau, essore le précipité formé, sèche à 60 °C sous pression réduite. On chromatographie les 426 mg de produit obtenu sur silice, élue par un mélange cyclohexane-acétate d'éthyle (6-4) et obtient 360 mg de produit. F = 260 °C.

On reprend ce dernier dans 8 cm3 de chlorure de méthylène, ajoute de l'éther isopropylique, concentre, essore et obtient 330 mg de produit attendu. F = 260 °C.

Spectre I.R. ($CHCl_3$)

C=O : 1645 $cm^{-1}$ - 1655 $cm^{-1}$
OH : 3600 $cm^{-1}$
aromatique + C=C conj : 1608 $cm^{-1}$ - 1600 $cm^{-1}$ - 1581 $cm^{-1}$ - 1510 $cm^{-1}$

Exemple 2 : 11béta, 17alpha-bis/4-(diméthylamino) phényl/ 17 béta-hydroxy estra-4,9-dièn-3-one.

Stade A : (1,2-éthanediyl) acétal cyclique de 5alpha, 17béta-dihydroxy 11béta, 17alpha-bis/4-(diméthylamino) phényl/ estra-9-èn-3-one (A) et (1,2-éthanediyl) acétal cyclique de 11béta, 17-bis/4-(diméthylamino) phényl/ 5alpha-hydroxy estra-9,16-dièn-3-one (B).

On sèche à 140 °C sous pression réduite 3,7 g de chlorure de cérium pendant deux heures, refroidit sous atmosphère inerte anhydre, ajoute 30 cm3 de tétrahydrofuranne et agite pendant 2 heures à température ambiante. On refroidit à 0 ° C, ajoute 25 cm3 d'une solution 0,77 M de bromure de /4-(diméthyl amino) phényl/ magnésium dans le tétrahydrofuranne, agite une heure à 0 °C et introduit 2,26 g de 3,3-(1,2-éthanediyl) acétal cyclique de 11béta-/4-(diméthylamino) phényl/ 5alpha hydroxy estra-4,9 dien- 3,17 dione dans 15 cm3 de tétrahydrofuranne anhydre puis laisse revenir à température ambiant Après une heure et demie, on verse le mélange réactionnel dans une solution de chlorure d'ammonium, ajoute de l'acétate d'éthyle, agite, filtre et extrait le filtrat à l'acétate d'éthyle. On lave la phase organique, sèche et concentre à sec. On chromatographie le résidu sur silice, élue par un mélange chlorure de méthylène - acétate d'éthyle (8-2) et isole après cristallisation dans l'éther 975 mg de produit attendu (A) F = 262 °C.

On joint les fractions restantes de la chromatographie aux liqueurs-mères de cristallisation et chromatographie à nouveau. On recueille 470 mg de produit A et 655 mg de produit (B) F = 214-216 °C.

Stade B : 11béta, 17alpha-bis/4-(diméthylamino) phényl/ 17 béta-hydroxy estra-4,9-dièn-3-one.

On dissout 865 mg de produit A dans 8 cm3 de méthanol et

4 cm3 d'acide chlorhydrique 2N et laisse réagir pendant une heure à température ambiante. On alcalinise sous agitation par addition d'une solution aqueuse de bicarbonate de sodium puis extrait au chlorure de méthylène. On lave la phase organique, sèche, concentre à sec et obtient 835 mg de produit brut.

Après recristallisation dans l'éther, puis dans le chlorure de méthylène et l'éther, on obtient le produit attendu fondant à 232 °C.

Spectre IR ($CHCl_3$)

OH libre : 3600 $cm^{-1}$
Bandes aromatiques : 1560 $cm^{-1}$ - 1518 $cm^{-1}$ - 1612 $cm^{-1}$ 3-céto : 1653 $cm^{-1}$.

Exemple 3 : 11béta, 17-bis/4-(diméthylamino) phényl/ estra-4,9,16-trièn-3-one.

On dissout 540 mg de (1,2-éthane diyl) acétal cyclique de 11béta, 17-bis/4-(diméthylamino) phényl/ 5alpha-hydroxy estra-9,16-dièn-3-one (exemple 2, stade A, produit B) dans 8 cm3 de méthanol avec 4 cm3 d'acide chlorhydrique 2N. Après une heure à température ambiante, on alcalinise avec une solution de bicarbonate de sodium, extrait au chlorure de méthylène, lave la phase organique, sèche et concentre à sec. On chromatographie le résidu sur silice, élue par un mélange chlorure de méthylène - acétate d'éthyle

(9-1) et obtient 400 mg de produit attendu.

Spectre I.R.

diènone : 1653 $cm^{-1}$ - 1600 $cm^{-1}$ - 863 $cm^{-1}$
diméthylaminophényle : 1611 $cm^{-1}$ et 1518 $cm^{-1}$

Exemple 4 : 11béta-/4-(diméthylamino) phényl/ 17béta-hydroxy 17alpha-(4-méthoxyphényl) estra-4,9-dièn-3-one.

Stade A : (1,2-éthanediyl) acétal cyclique de 5alpha, 17 béta-dihydroxy 11béta-/4-(diméthylamino) phényl/ 17alpha-/4-méthoxyphényl/ estr-9-èn-3-one (produit A), et son isomère 17béta-aryle (produit B).

On opère comme au stade A de l'exemple 2 à partir de 12 cm3 d'une solution 0,9 M de bromure de 4-méthoxy phényl magnésium dans le tétrahydrofuranne. On chromatographie sur silice le résidu d'extraction, élue par un mélange cyclohexane-acétate d'éthyle (8-2) et isole 980 mg de produit A F 209°C après recristallisation dans le mélange chlorure de méthylène - éther isopropylique et 609 mg de produit B F 264°C après recristallisation dans le méthanol.

Stade B : 11béta-/4-(diméthylamino) phényl/ 17béta-hydroxy 17alpha-(4-méthoxy phényl) estra 4,9-dièn-3-one.

On dissout 1,54 g de produit A dans 35 cm3 de méthanol, ajoute 5 cm3 d'acide chlorhydrique 2N, laisse réagir pendant une heure et demie et verse le mélange réactionne l dans une solution aqueuse de bicarbonate de sodium. On extrait à l'acétate d'éthyle, lave à l'eau la phase organique, sèche et amène à sec. On chromatographie le résidu sur silice en éluant par le mélange chlorure de méthylène - acétate d'éthyle (9-1) puis (8-2) et obtient 1,38 g de produit attendu. F = 266°C après recristallisation dans le mélange éther - éther isopropylique.

Spectre IR ($CHL_3$)

OH : 3600 $cm^{-1}$
C = 0 : 165 $cm^{-1}$
Aromatiques : 1611 $cm^{-1}$ - 1560 $cm^{-1}$ - 1517 $cm^{-1}$

Exemple 5 : 11béta-/4-(diméthylamino) phényl/ 17-(4-méthoxy phényl) estra-4,9,16-trièn-3-one.

On dissout 680 mg de produit B obtenu à l'exemple 4 stade A dans 20 cm3 de méthanol et 2,5 cm3 d'acide chlorhydrique 2N. Après une heure et demie à température ambiante, on dilue à l'eau, alcalinise avec du bicarbonate de sodium et extrait au chlorure de méthylène. On lave à l'eau la phase organique, sèche et amène à sec et obtient un mélange de produit attendu et de produit 17alpha méthoxy 17béta-(4-méthoxy phényl)

On dissout le résidu dans 4 cm3 de tétrahydrofuranne et 2 cm3 d'acide chlorhydrique 2N et laisse réagir pendant 3 heures. On ajoute de l'eau, du bicarbonate de sodium et extrait au chlorure de méthylène. On lave la phase organi que, sèche et obtient 603 mg de produit attendu.
$/\alpha/_D$ +247° (c = 1% $CHCl_3$)

Spectre IR ($CHCl_3$)

C = 0 en 3 : 1654 $cm^{-1}$
Aromatiques : 1611 $cm^{-1}$ - 1569 $cm^{-1}$ - 1517 et 1510 $cm^{-1}$

Exemple 6 : 11béta-/4-(diméthylamino) phényl/ 17béta-hydroxy 17alpha-(4-hydroxyphényl) estra-4,9-dièn-3-one (produit A) et 11béta-/4-(diméthylamino) phényl/ 17-(4-hydroxy phényl) estra-4,9,16-trièn-3-one (produit B).

D'abord, on opère comme au stade A de l'exemple 2 en utilisant 20 cm3 d'une solution 0,72M de bromure de /4-(2'-tétrahydropyranyloxy) phényl/ magnésium dans le tétrahydrofuranne. Ensuite, on verse le mélange réactionnel dans une so lution glacée renfermant 100 cm3 d'eau et 5 cm3 d'acide acétique, extrait à l'acétate d'éthyle, lave la phase organique avec une solution de bicarbonate de sodium puis à l'eau, sèche et concentre à sec. On dissout le résidu dans 20 cm3 de méthanol, ajoute 5 cm3 d'acide

chlorhydrique 2N, agite une heure et demie puis verse le mélange dans une solution aqueuse de bi carbonate de sodium et extrait au chlorure de méthylène. On évapore à sec la phase organique, chromatographie le résidu sur silice, élue par un mélange chlorure de méthylène-acétate d'éthyle (8-2) et recueille 720 mg de produit B et 730 mg de produit A. On chromatographie à nouveau le produit B (720 mg) sur silice (éluant : benzène -acétate d'éthyle 7-3) et obtient 495 mg de produit B.
$/\alpha/_D$ = + 240° ± 3° (c = 1% dans $CHCl_3$).

On chromatographie une nouvelle fois le produit A (730 mg) sur silice, élue par un mélange benzène-acétate d'éthyle (6-4) et recueille 540 mg de produit A que l'on recristallise dans l'acétate d'éthyle puis dans l'isopropanol. On obtient 463 mg de produit A solvaté. F = 208°C.
$/\alpha/_D$ + 133° (c = 1% dans $CHCl_3$)

Spectre IR ($CHCl_3$)

OH : 3596 $cm^{-1}$ et associé
3-céto : 1651 $cm^{-1}$
aromatiques : 1612 $cm^{-1}$ - 1594 $cm^{-1}$ - 1562 $cm^{-1}$

Exemple 7 : 17alpha /4-(diméthylamino) phényl/ 17béta-hydroxy 11béta-/4-(méthylthio) phényl/ estr-9-èn-3-one.

Stade A :
3,3-(1,2-éthanediyl) acétal cyclique de 5alpha-hydroxy 11béta-/4-(méthylthio) phényl/ estr-9-en-3,17-dione.

On refroidit à -6°C, 0,672 g de chlorure cuivrique, 0,212 g de chlorure de lithium, 165 cm3 de tétrahydrofuranne et 16,5 g de 3,3-(1,2-éthanediyl) acétal cyclique de 5alpha,10alpha-époxy estra-9(11)-èn-17-one et introduit, goutte à goutte en une heure 15 minutes, 100 cm3 d'une solution 0,75M de bromure de [(4-méthylthio) phényl] magnésium et agite à -10°C sous atmosphère inerte pendant encore une heure. On ajoute 100 cm3 d'une solution saturée en chlorure d'ammonium, agite 10 minutes, extrait à l'acétate d'éthyle, lave à l'eau, sèche et concentre à sec sous pression réduite. On reprend le résidu par un peu d'hexane, essore le produit cristallisé, lave à l'hexane, sèche et obtient 22,2 g de produit brut attendu. F = 202°C.

On purifie 2 g de celui-ci par chromatographie sur silice (éluant : cyclohexane-acétate d'éthyle (1-1) à 1°/°° de triéthylamine puis par cristallisation dans l'acétate d'éthyle et obtient 0,928 g de produit attendu. F = 209°C.

Stade B : (1,2-éthanediyl) acétal cyclique de 5alpha-17béta-dihydroxy 17alpha - /4-(diméthylamino) phényl/ 11béta-/4-(méthylthio) phényl/ estr-9-èn-3-one (produit A). (1,2-éthanediyl)-acétal cyclique de 5alpha,17alpha-dihydroxy 17béta-/4-(diméthylamino) phényl/ 11béta/4-(méthylthio) phényl/ estr-9-èn-3-one (produit B).

On déshydrate à 150 C sous pression de 1mmHg pendant deux heures 7,7 g de chlorure de cérium, ajoute en 10 minutes à 5°C, 35 cm3 d'une solution 0,87M de bromure de [4-(diméthyl amino) phényl magnésium et agite cette suspension pendant une heure à 0-5°C. On introduit 5 g de produit brut obtenu ci-dessus (F= 202°C) en solution dans 40 cm3 de tétrahydrofuranne anhydre et laisse revenir à température ambiante. On ajoute, à nouveau, 12 cm3 de solution de magnésien et laisse une nuit à température ambiante.

On verse le mélange réactionnel dans 200 cm3 de solution aqueuse à 10 % de chlorure d'ammonium, décante, lave à l'eau salée, extrait à l'acétate d'éthyle, sèche et amène à sec. On chromatographie le résidu sur silice, élue sous pression par un mélange : chlorure de méthylène-acétate d'éthyle (7-3) à 1% de triéthylamine et obtient 1,97 g de produit A (Rf. = 0,44) et 1,08 g de produit B (Rf. = 0,21).

Spectre I.R. $CHCl_3$ pour A et B

5-OH : 3510 $cm^{-1}$
17-OH : 3598 $cm^{-1}$

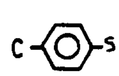 :

1491 $cm^{-1}$

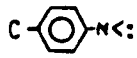

1611 - 1559 - 1520 $cm^{-1}$

Stade C : 17alpha-/4-(diméthylamino) phényl/ 17béta-hydroxy 11bèta-/4-(méthylthio) phényl/estra-4,9-dien-3-one

On dissout 1,94 g de produit A obtenu ci-dessus dans 20 cm3 de tétrahydrofuranne, ajoute 5 cm3 d'acide chlorhydrique 2N et agite 50 minutes. On verse le mélange dans une solution de bicarbonate de sodium, extrait à l'acétate d'éthyle, lave à l'eau, sèche et amène à sec.

On chromatographie le résidu sur silice en éluant par un mélange : chlorure de méthylène - acétate d'éthyle (8-2) et obtient 1,24 g de produit attendu. On dissout ce dernier dans 10 cm3 de chlorure de méthylène, ajoute à chaud de l'éther isopropylique, concentre, glace, essore et obtient 1,055 g de produit attendu cristallisé F = 236 ° C

$/\alpha/_D$ = +75 ° ± 1,5 ° (c = 1% $CHCl_3$)

Exemple 8 : 17béta-/4-(diméthylamino) phényl/17alpha-hydroxy 11béta-/(4-méthylthio) phényl/estra 4,9-dièn-3-one (produit A) 17-/4-(diméthylamino) phényl/ 11béta-/(4-méthylthio) phényl/ estra-4,9,16-trièn-3-one (produit B).

On dissout 1,03 g de (1,2-éthanediyl) acétal cyclique de 5alpha,17alpha-dihydroxy 17béta-/4-(diméthylamino) phényl/11 béta-/4-(méthylthio) phényl/ estr-9-èn-3-one obtenu à l'exemple 7 stade B, dans 10 cm3 de tétrahydrofuranne puis ajoute 5,4 cm3 d'acide chlorhydrique N, agite 30 minutes et verse le mélange sur 20 cm3 d'une solution de bi carbonate de sodium.

On extrait à l'acétate d'éthyle, lave à l'eau salée, sèche et amène à sec. On chromatographie le résidu sur silice en éluant par un mélange cyclohexanne - acétate d'éthyle (7-3) à 1 °/₀₀ de triéthylamine et obtient 470 mg de produit B et 115 mg de produit A.

On dissout ce dernier dans 4 cm3 de chlorure de méthylène, ajoute 10 cm3 d'éther isopropylique, concentre, glace, essore et obtient 95 mg de produit A. F = 222 ° C.

Spectre IR ($CHCl_3$) de A

OH libre : 3597 $cm^{-1}$
C=0 : 1652 $cm^{-1}$

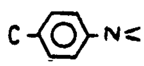 :

1613 $cm^{-1}$ - 1558 $cm^{-1}$ - 1521 $cm^{-1}$

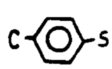 :

1492 $cm^{-1}$

Produit B

$/\alpha/_D$ = + 178,5 ° ± 2,5 ° (c = 1% $CHCl_3$)

Exemple 9 : 11béta-/4-(diméthylamino) phényl/17béta-hydroxy 17 alpha (4-méthyl phényl) estra-4,9-dièn-3-one (produit A) 11 béta-/4-(diméthylamino) phényl/17alpha-hydroxy 17béta (4-méthyl phényl) estra-4,9-dièn-3-one (produit B) et 11béta-/4-(diméthylamino) phényl/ 17alpha-méthoxy 17béta-(4-méthyl phéyl) estra-4,9-dièn-3-one (produit C).

Stade A : (1,2-éthanediyl) acétal cyclique de 5alpha,17béta-dihydroxy 11béta-/4-(diméthylamino) phényl/ 17alpha-(4-méthyl phényl) estra-9-èn-3-one et son isomère 17béta-(4-méthyl phényl).

Préparation du magnésien.

On chauffe à 50 °C, 1,45 g de tournures de magnésium, 2 cm3 d'une solution contenant 8,5 g de parabromotoluène dans 44 cm3 de tétrahydrofuranne anhydre. Après amorcage de la réaction, on ajoute en une heure le reste de la solution puis agite encore 45 minutes à 50 °C et obtient une solution du magnésien attendu titrant 0,89N.

Condensation.

On opère comme au stade A de l'exemple 2 en utilisant 18 cm3 de la solution de magnésien préparée ci-dessus.

On verse le mélange réactionnel dans une solution glacée d'acide acétique à 4% d'eau, extrait à l'acétate d'éthyle, lave à l'eau la phase organique, filtre un insoluble, sèche sous pression réduite et obtient 451 mg du produit 17béta-(4-méthyl phényl) F = 305 °C.

On sèche le filtrat, évapore à sec et obtient 1,8 g du mélange des produits 17alpha et 17béta-(4-méthylphényl) attendu.

Stade B : 11béta-/4-(diméthylamino)phényl/ 17béta-hydroxy 17alpha-(4-méthyl phényl) estra-4,9-dièn-3-one (produit A), 11béta-/4-(diméthylamino) phényl/ 17alpha-hydroxy 17béta-(4-méthyl phényl) estra-4,9-dièn-3-one (produit B) et 11béta-/4-(diméthylamino)phényl/ 17alpha-méthoxy 17béta-(4-méthyl phényl) estra-4,9-dièn-3-one (produit C).

1) On dissout 1,8 g du mélange obtenu au stade A dans 10 cm3 d'acide chlorhydrique 2N, agite pendant deux heures sous atmosphère inerte et verse dans une solution aqueuse de bicarbonate de sodium. On extrait à l'acétate d'éthyle, lave avec une solution aqueuse saturée en chlorure de sodium, sèche la phase organique et évapore à sec. On chromatographie le résidu sur silice, en éluant par un mélange chlorure de méthylène - acétate d'éthyle (9-1) et sépare 195 mg de produit C et 672 mg de produit A.

On recristallise le produit A dans un mélange chlorure de méthylène - éther isopropylique et obtient 550 mg de produit A cristallisé F = 262 °C.

$/\alpha/_D$ = +167 ° ± 2,5 ° (c = 1% $CHCl_3$)

2) On dissout 445 mg des 451 mg de produit 17béta-(4-méthyl phényl) obtenus ci-dessus au stade A dans 5 cm3 de méthanol, ajoute 1 cm3 d'acide chlorhydrique 2N, agite une heure à température ambiante et verse le mélange réactionnel dans une solution aqueuse de bi carbonate de sodium. On extrait à l'acétate d'éthyle, lave à l'eau, sèche et amène à sec. On chromatographie sur silice le résidu en éluant par un mélange chlorure de méthylène - acétate d'éthyle (8-2) et sépare 130 mg de produit C et 220 mg de produit B. On recristallise ce dernier dans un mélange chlorure de méthylène - éther isopropylique puis dans un mélange chlorure de méthylène - acétate d'éthyle (1-1) et obtient 146 mg de produit B cristallisé. F = 304 °C

On cristallise les 195 mg et 130 mg de produit C obtenus ci-dessus dans un mélange chlorure de méthylène - éther isopropylique et obtient 227 mg de produit C cristallisé. F = 238 °C.

$/ /_D$ = +170 ° ± 2,5 ° (c = 1% $CHCl_3$) Exemple 10 : 11béta-/4-(diméthylamino)phényl/ 17béta-hydroxy 17alpha-(2-hydroxyphényl) estra 4,9-dièn 3-one (produit A)

11béta-/4-(diméthylamino) phényl/ 17alpha-hydroxy 17béta-(2-hydroxy phényl) estra 4,9-dièn-3-one (produit B).

Stade A : (1,2-éthane diyl) acétal cyclique de 5alpha,17béta-dihydroxy 11béta-/4-(diméthylamino) phényl/ 17alpha-(2-hydroxy phènyl) estr-9-èn-3-one et son isomère 17béta-(2-hydroxy phényl).

Préparation du magnésien.

On opère comme à l'exemple 9 à partir de 12,9 g de l'éther tétrahydropyrannylique du 2-bromophénol en chauffant à 55 °-60 °C et obtient la solution du magnésien attendu titrant 0,67N.

Condensation.

On opère comme à l'exemple 2 stade A en utilisant 40 cm3 de la solution préparée ci-dessus en chauffant à 40 ° C pendant 5 heures.

Après extraction, évaporation du solvant, on chromatographie le résidu sur silice, élue par le mélange cyclohexane - acétate d'éthyle (1-1) et obtient 973 mg de composé 17 alpha-(2-hydroxyphényl) et 595 mg d'un mélange contenant l'isomère 17béta-(2-hydroxy phényl) et le 17béta/(2-tétrahydropyrannyloxy) phényl/ correspondant.

On recristallise les 973 mg de produit 17alpha-(2-hydroxy-phényl) dans le mélange chlorure de méthylène - éther isopropylique et obtient 758 mg de produit cristallisé F = 287 ° C.

Stade B : 11bèta-/4-(diméthylamino) phényl/17béta-hydroxy 17alpha-(2-hydroxyphényl) estra-4,9-dièn-3-one (produit A). 11béta-/4-(diméthylamino) phényl/17alpha-hydroxy 17béta-(2-hydroxy phényl) estra-4,9-dièn-3-one (produit B).

1) On dissout 1,27 g du produit 17 alpha-(2-hydroxyphényl) obtenu comme ci-dessus dans 13 cm3 de tétrahydrofuranne, ajoute 6,5 cm3 d'acide chlorhydrique N, laisse à température ambiante pendant 45 minutes puis verse le mélange dans une ambiante pendant 45 minutes puis verse le mélange dans une solution aqueuse de bicarbonate de sodium. On extrait à l'acétate d'éthyle, lave à l'eau, sèche, évapore à sec, chromatographie le résidu sur silice en éluant par un mélange cyclohexane - acétate d'éthyle (6-4) et isole 847 mg de produit A attendu.

Après cristallisation dans le mélange chlorure de méthylène - éther isopropylique, on obtient 780 mg de produit A fondant à 266 ° C.

$/\alpha/_D$ = + 185 ° ± 2,5 ° (c = 1% $CHCl_3$)

2) On dissout 170 mg du mélange obtenu au stade A dans 2 cm3 de tétrahydrofuranne, ajoute 1,5 cm3 d'acide chlorhydrique N et laisse en contact pendant une heure. On verse le mélange réactionnel sur une solution aqueuse de bi carbonate de sodium, extrait à l'acétate d'éthyle, lave à l'eau, sèche et concentre à sec. On recristallise le résidu dans un mélange chloroforme - éther isopropylique puis chloroforme - méthanol et obtient 105 mg de produit B. F = 275 ° C.

$/\alpha/_D$ = + 112 ° ± 2,5 ° (c= 0,46% $CHCl_3$)

Exemple 11 : 11béta-/4-(diméthylamino) phényl/17béta-hydroxy 17alpha-(2-méthoxyphényl) estra-4.9-dièn-3-one (produit A), 11béta-/4-(diméthylamino) phényl/ 17alpha-hydroxy 17béta-(2-méthoxy phényl) estra-4,9-dièn-3-one (produit B).

Stade A : (1,2-éthanediyl) acétal cyclique de 5alpha, 17béta-dihydroxy 11béta-/4-(diméthylamino)phényl/ 17alpha-(2-méthoxy phényl) estr-9-èn-3-one et (1,2-éthanediyl) acétal cyclique de 5alpha,17alpha-dihydroxy11béta-/4-(diméthylamino) phényl/17 béta-(2-méthoxyphényl) estr-9-èn-3-one.

On opère comme à l'exemple 4 stade A à partir de 25 cm3 d'une solution 0,8N de bromure de (2-méthoxyphényl) magnésium. Après chromatographie sur silice du résidu d'extraction en éluant par du cyclohexane puis par un mélange acétate d'éthyle - cyclohexane (6-4) on recueille 806 mg de produit 17alpha-(2-méthoxy phényl) et 1,44 g de l'isomère 17béta-(2-méthoxy phényl).

On recristallise les 806 mg dans le mélange chlorure de méthylène - éther isopropylique et obtient 738 mg de produit pur. F = 242-244 ° C.

Stade B : 11béta-/4-(diméthylamino) phényl/17béta-hydroxy 17alpha-(2-méthoxy phényl) estra-4,9-dièn-3-one (produit A) 11béta-/4-(diméthylamino) phényl/ 17alpha-hydroxy 17béta-(2-méthoxy phényl) estra-4,9-dièn-3-one (produit B).

1) On dissout 588 mg de produit 17alpha-(2-méthoxyphényl) obtenu au stade A dans 8 cm3 de tétrahydrofuranne, ajoute 3,15 cm3 d'acide chlorhydrique N et maintient à température ambiante pendant 45 minutes.

On verse le mélange réactionnel dans une solution aqueuse de bi carbonate de sodium, extrait à l'acétate d'éthyle, lave à l'eau, sèche et concentre à sec sous pression réduite. On chromatographie le résidu sur silice en éluant par un mélange cyclohexane-acétate d'éthyle (4-6) et obtient 390 mg de produit A. On dissout ce dernier dans 20 cm3 d'acétate d'éthyle au reflux, filtre, concentre sous pression réduite, refroidit, essore et obtient 297 mg de produit A. F = 264 ° C.

$/\alpha/_D$ = + 198 ° ± 3 ° (c = 1% $CHCl_3$)

2) On opère comme ci-dessus en utilisant 1,4 g de produit 17béta-(2-méthoxy phényl) obtenu au stade A (éluant de chromatographie cyclohexane - acétate d'ethyle 1-1) et obtient 732 mg de produit B après recristallisation dans l'acétate d'éthyle F = 222 ° C.

$/\alpha/_D$ = +163 ° ± 2,5 ° (c = 1% $CHCl_3$)

Exemple 12 : 11béta-/4-(diméthylamino) phényl/17béta-hydroxy 17alpha-(3-méthoxy phényl) estra 4,9-dièn-3-one (produit A) 11béta-/4-(diméthylamino) phényl/17alpha-hydroxy 17béta-(3-méthoxy phényl) estra-4,9-dièn-3-one (produit B).

Stade A : (1,2-éthanediyl) acétal cyclique de 5alpha,17béta-dihydroxy 11béta-/4-(diméthylamino) phényl/ 17alpha-(3-méthoxyphényl) estr-9-èn-3-one et (1,2-éthanediyl) acétal cyclique de 5alpha,17alpha-dihydroxy 11béta-/4-(diméthylamino) phényl/17béta-(3-méthoxy phényl) estr-9-èn-3-one.

On opère comme à l'exemple 11 à partir de 9,44 g debromure de (3-méthoxy phényl) magnésium et obtient 600 mg de produit 17alpha-/3-méthoxy phényl). F = 220°C et 1,104 g de l'isomère 17béta-(3-méthoxy phènyl). F = 252°C.

Stade B : 11béta-/4-(diméthylamino) phényl/17béta-hydroxy 17alpha-(3-méthoxy phényl) estra-4,9-dièn-3-one (produit A) 11béta-/4-(diméthylamino) phényl/17alpha-hydroxy 17béta-(3-méthoxy phényl) estra 4,9-dièn-3-one (produit B).

1) On opère comme au stade $B_1$ de l'exemple 11 en utilisant 594 mg du produit 17alpha-(3-méthoxy phényl) obtenu ci-dessus et obtient 337 mg de produit A.
$/\alpha/_D$ = + 137° ± 2° (c = 0,9% $CHCl_3$)

2) On opère comme au stade $B_2$ de l'exemple 11 en utilisant 1,21 g du produit 17béta-(3-méthoxy phényl) obtenu au stade A, chromatographie le résidu d'extraction sur silice en éluant avec un mélange hexane - acétate d'éthyle (1-1), recristallise dans l'acétate d'éthyle et obtient 698 mg de produit B. F = 262°C.

Exemple 13 : 11béta-/4-(diméthylamino) phényl/17béta-hydroxy 17alpha-(3-hydroxyphényl) estra-4,9-dièn-3-one (produit A) 11béta-/4-(diméthylamino) phényl/17alpha-hydroxy 17béta-(3-hydroxy phényl) estra-4,9-dièn-3-one (produit B).

Stade A : (1,2-éthanediyl) acétal cyclique de 5alpha,17béta-dihydroxy 11béta-/4-(diméthylamino) phényl/17alpha-/3-(tétrahydro 2H-2-pyrannyloxy)phényl/ estr-9-èn-3-one et (1 ,2-éthanediyl) acétal cyclique de 5alpha,17alpha-dihydroxy 11béta-/4- (diméthylamino)phényl/ 17béta-/3-(tétrahydro 2H-2-pyrannyloxy) phényl/ estra-9-èn-3-one.

On opère comme à l'exemple 10 à partir de 12,9 g d'éther tétrahydropyrannylique de 3-bromophénol.

On chromatographie sur silice le résidu sec obtenu après extraction en éluant par un mélange chlorure de méthylène-acétate d'éthyle - triéthylamine (80-20-1) et obtient 830 mg de produit 17alpha-arylé et 1,55 g de 17béta-arylé qui, recristallisé dans l'acétate d'éthyle, fond à 224°C.

Spectre IR sur 17alpha aryle ($CHCl_3$)

OH en 17 : 3604 $cm^{-1}$
OH en 5 : 3510 $cm^{-1}$
Aromatiques 1610 $cm^{-1}$ - 1517 $cm^{-1}$ - 1562 $cm^{-1}$ - 1580 $cm^{-1}$

Stade B :

11béta-[4-(diméthylamino) phényl] 17béta-hydroxy 17alpha-(3-hydroxy phényl) estra-4,9-diène-3-one (produits A) 11béta [4-diméthylamino) phényl] 17 alpha-hydroxy 17béta-(3-hydroxy phényl) estra-4,9-dièn-3-one (produitB)

On dissout 890 mg de produit 17alpha-arylé obtenu au stade A, dans 9 cm3 de tétrahydrofuranne, ajoute 4,5 cm3 d'acide chlorhydrique 2N et agite une heure et demie à température ambiante.

On verse le mélange réactionnel dans une solution aqueuse de bicarbonate de sodium, extrait à l'acétate d'éthyle, lave à l'eau salée et évapore à sec. On chromatographie le résidu sur silice en éluant par un mélange cyclohexanne - acétate d'éthyle 1-1 et obtient 554 mg de produit attendu.
$/\alpha/_D$ = + 138° ± 2° (c = 1% $CHCl_3$)

On opère comme ci-dessus avec 800 mg de produit 17béta-arylé obtenu au stade A, 8 cm3 d'acide chlorhydrique 2N et recristallise le résidu d'extraction dans un mélange méthanol - acétate d'éthyle. On obtient 500 mg de produit attendu.
$/\alpha/_D$ = + 163° ± 3° (c = 0,33% $CHCl_3$)

Exemple 14 : 11béta-/4-(diméthylamino) phényl/ 17alpha-phényl estra-1,3.5(10)-trién-3,17béta-diol et son isomère 17béta-phényl.

Stade A : 11béta-/4-(diméthylamino) phényl/estra-4,9-diène 3,17-dione.

On agite à température ambiante pendant une heure, 1 g de 3,3-(1,2-éthanediyl)acétal cyclique de 11béta-/4-(diméthylami-no) phényl/ 5alpha-hydroxy estr-9-èn-17-one, 30 cm3 d'éthanol et 5 cm3 d'acide chlorhydrique 2N.

On neutralise le mélange par de la triéthylamine puis concentre à faible volume. On extrait au chlorure deméthylène, sèche la phase organique, évapore à sec le solvant. On chromatographie le résidu sur silice en éluant par un mélange chlorure de méthylène - acétone 95-5 et recueille 588 mg de produit attendu. Celui-ci est repris dans 2 cm3 d'éther. On sépare ainsi 510 mg de produit. F = 166°C.

Stade B : 11béta-/4-(diméthylamino) phényl/ 3-hydroxy estra 1,3,5(10)-trièn-17-one.

On dissout 8,9 g du produit obtenu comme précédemment dans 225 cm3 de méthanol, ajoute 8,9 g d'hydroxyde de palladium sur magnésie et porte au reflux pendant une heure. On filtre, évapore à sec le filtrat, chromatographie sur silice le résidu en éluant par un mélange éther - acétate d'éthyle (1-1) + 1% de triéthylamine. On recueille 8,9 g de résine que l'on cristallise dans un mélange acétate d'éthyle - éther isopropylique et isole 5,1 g de produit attendu. F = 256 °C.

Stade C : 11béta-/4-(diméthylamino) phényl/ 17alpha-phényl estra-1,3,5(10)-trièn-3,17béta-diol et son isomère 17béta-phényl.

Préparation du magnésien

A 1,45 g de tournures de magnésium, on ajoute 1 cm3 d'une solution de 5,25 cm3 de bromobenzène dans 50 cm3 de tétrahydrofuranne anhydre. On chauffe à 50 °C et après amorcage de la réaction, introduit, goutte à goutte, le reste de la solution de bromobenzène en 45 minutes de facon à maintenir la température à 60 °C. On agite encore pendant 30 minutes à 60 °C. La solution obtenue titre 0,9N.

Condensation

On chauffe à 140 °C sous pression réduite pendant 2 heures 2 g de chlorure de cérium hydraté.
On ajoute à température ambiante 6 cm3 de tétrahydrofuranne et agite 2 heures. On refroidit à 0 °C puis ajoute 10 cm3 de la solution préparée ci-dessus. On agite deux heures à 0 ° C et introduit 700 mg de produit obtenu au stade B en solution dans 8 cm3 de tétrahydrofuranne et agite une heure à 0 ° C sous atmosphère inerte. On verse alors dans une solution aqueuse de chlorure d'ammonium et extrait à l'acétate d'éthyle.

On lave à l'eau la phase organique, sèche, évapore à sec sous pression réduite. On reprend le rési du par le chlorure de méthylène, ajoute de l'éther isopropylique puis isole 540 mg de produit impur que l'on recristallise dans l'éthanol pour obtenir 266 mg de 11béta-/4-(diméthylamino) phényl/ 17alpha-phényl estra-1,3,5(10)-trièn-3,17béta-diol. F = 277 °C.

On chromatographie sur silice les liqueurs-mères de cristallisation en éluant par un mélange toluène - acétate d'éthyle (8-2) et obtient 80 mg de 11béta-/4-(diméthylamino) phényl/ 17béta-phényl estra-1,3,5(10)-trien-3,17alpha-diol que l'on recristallise dans l'acétate d'éthyle/éther isopropylique. On isole 73 mg de cristaux. F 120 °C.

Isomère alpha-phényl

$/\alpha/_D$ = -286 ° ± 3,5 ° (c = 1% $CHCl_3$)

Spectre IR ($CHCl_3$)

OH : 3597 $cm^{-1}$
Aromatique : 1615 $cm^{-1}$ - 1580 $cm^{-1}$ - 1559 $cm^{-1}$ - 1520 $cm^{-1}$ - 1500 $cm^{-1}$

Isomère béta-phényl

Spectre IR ($CHCl_3$)
OH : 3599 $cm^{-1}$
Aromatique : 1614 $cm^{-1}$ - 1583 $cm^{-1}$ - 1560 $cm^{-1}$ - 1520 $cm^{-1}$ 1498 $cm^{-1}$

Exemple 15 : 11béta,17alpha-bis/4-(diméthylamino) phényl/estra-1 3,5(10)-trien-3,17béta-diol.

On opère comme à l'exemple 14, stade C, en utilisant 33 cm3 d'une solution 1N de bromure de 4-(diméthylamino) phényl magnésium, 2 g du produit obtenu au stade B de l'exemple 14 dans 40 cm3 de tétrahydrofuranne et on agite une heure à +3 ° C. On verse le mélange réactionnel sur une solution aqueuse de chlorure d'ammonium.

D'une part, on essore un insoluble que l'on reprend par un mélange chlorure de méthylène - éthanol 1-1, chauffe au reflux, essore et recueille 1,57 g de produit attendu. F = 206 °C.

D'autre part, on extrait le filtrat à l'acétate d'éthyle, lave, sèche et évapore à sec la phase organique, chromatographie sur silice le résidu en éluant par un mélange chlorure de méthylène - acétate d'éthyle (9-1) et obtient 420 mg de produit attendu.

On recristallise les deux lots de produit dans un mélange éthanol - chloroforme et obtient 1,8 g de produit. F = 208 ° C.
/α / $_D$ = -314 ° ± 4 ° (c = 1% pyridine).

Exemple 16 : 11béta-[4-(diméthylamino) phényl] 17alpha-(4-hydroxyphényl) estra 1,3,5 (10)-triène-3,1èbéta-diol.

Stade A : 11béta-[4-(diméthylamino) phényl] 17alpha-4-tétrahydro 2H-2-pyrannyloxy) phényl] estra-1,3,5 (10)-triène-3,17béta-diol.

Préparation du magnésien :

On opère comme indiqué à l'exemple 9 à partir de 8,3 g de 4-[2'-RS-tétrahydropyrannyloxy] 1-bromobenzène et 1 g de magnésien. On obtient la solution attendue titrant 0,85 N.

Condensation :

On opère comme au stade A de l'exemple 2 en utilisant 2,5 g de chlorure de cérium dans 12 cm3 de tétrahydrofuranne, 18 cm3 de la solution de magnésien préparée ci-dessus, et 0,97 g de produit obtenu comme au stade B de l'exemple 14. On obtient après concentration 5 g de produit brut que l'on chromatographie sur silice (éluant : éther de pétrole Eb : 40 ° -70 ° C-acétate d'éthyle 7-3). On recueille 0,332 g de produit attendu.
Stade B : 11béta-[4-(diméthylamino) phényl] 17alpha-(4-hydroxyphényl) estra 1,3,5 (10)-triène-3,17béta-diol.
On opère comme au stade B de l'exemple 2 à partir de 0,307 g de produit obtenu au stade A dans 6 cm3 de méthanoil et 0,6 cm3 d'acide chlorhydrique 2N. On obtient 0,205 g de produit attendu (F > 360 ° C) que l'on recristallise dans un mélange chlorure de méthylène-éthanol (1-1).
[α]$_D$ = 323,5 ° ± 3,5 ° (c = 1% pyridine).

| Analyse : $C_{32}H_{37}NO_3$ : 483,65 | | | | | | |
|---|---|---|---|---|---|---|
| Calculé<br>Trouvé | C% | 79,47<br>79,3 | H% | 7,71<br>7,9 | N% | 2,89<br>2,8 |

Exemple 17 : 11béta-[4-(diméthylamino) phényl] 17-(4-hydroxy-phényl) estra 1,3,5 (10), 16-tétraèn-3-ol.

On opère comme au stade B de l'exemple 2 en utilisant 0,15 g du produit obtenu au stade B de l'exemple 16 dans 3 cm3 de méthanol et 0,31 cm3 d'acide chlorhydrique 12N. On obtient 0,166 g de produit brut que l'on purifie par chromatographie sur silice (éluant : éther de pétrole Eb. 40 ° -70 ° C-acétate d'éthyle 1-1).

| Analyse sur produit séché à 150 ° C : $C_{32}H_{35}NO_2$ : 465,65 | | | | | | |
|---|---|---|---|---|---|---|
| Calculé<br>Trouvé | C% | 82,54<br>81,4 | H% | 7,57<br>7,6 | N% | 3,00<br>2,9 |

Exemple 18 : 11béta-[4-[2-(diméthylamino) éthoxy] phényl] 17béta-hydroxy 17alpha (4-hydroxyphényl) estra-4,9-diène-3-one.

Stade A : (1,2-éthanediyl) acétal cyclique de 5alpha, 17béta-dihydroxy 11béta-[4-[2-(diméthylamino) éthoxy] phényl] 17alpha-[4-(tétrahydro2H-2-pyrannyloxy) phényl] estr-9-èn-3-one et (1,2-éthanediyl) acétal cyclique de 5alpha, 17alpha-dihydroxy 11béta-[4-[2-(diméthylamino) éthoxy] phényl] 17béta-[4-(tétrahydro2H-2-pyrannyloxy) phényl] estr-9-èn-3-one.
On opère comme au stade A de l'exemple 2 en utilisant au départ 7,74 g de chlorure de cérium dans 30 cm3 de tétrahydrofuranne, 30 cm3 de la solution de bromure de [4-(tétrahydropyrannyl) oxy phényl] de magnésium préparée comme à l'exemple 16 puis 2,05 g de (1,2-éthanediyl) acétal cyclique de 5alpha-hydroxy 11béta-[4-[2-diméthylamino) éthoxyl phényl] estr-9-èn-3,17-dione préparée à l'exemple 13 du brevet européen 0 097 572. On obtient 8,2 g de produit attendu sous forme de mélange que l'on chromatographie sur silice (éluant : acétate d'éthyle à 4% de triéthylamine), on récupère 1,03 g du composé 17alpha-hydroxy. Après chromatographie sur silice du résidu (éluant : méthanol-solution aqueuse d'acétate d'ammonium (0,05M) 8-2), on recueille 1,34 g du composé 17béta-hydroxy.

Stade B : 11béta-[4-[2-(diméthylamino) éthoxyl phényl] 17béta-hydroxy 17alpha (4-hydroxyphényl) estra-4,9-dièn-3-one.

On opère comme au stade B de l'exemple 2 en utilisant au départ 1,25 g du composé 17béta-hydroxy obtenu au stade précédent dans 13 cm3 de méthanol et 0,95 cm3 d'acide chlorhydrique 2N. On obtient 0,968 g de produit brut que l'on purifie par cristallisation dans le méthanol. F ≃ 160 °C.

$[\alpha]_D$ = +87° ± 1,5° (c = 1% chloroforme).

| Analyse sur produit séché à 120 °C : $C_{34}H_{41}NO_4$ : 527,71 | | | | | | |
|---|---|---|---|---|---|---|
| Calculé<br>Trouvé | C% | 77,38<br>77,3 | H% | 7,83<br>7,6 | N% | 2,65<br>2,6 |

Exemple 19 : 11béta-[4-[2-(diméthylamino) éthoxy] phényl] 17-(4-hydroxyphényl) estra-4,916-trièn-3-one.

METHODE A :

On dissout 0,629 g du composé 17alpha-hydroxy obtenu au stade A de l'exemple 18 dans 3 cm3 de tétrahydrofuranne, ajoute 1,5 cm3 d'acide chlorhydrique 2N et agite 30 minutes. On verse le mélange dans une solution de bi carbonate de sodium, extrait à l'acétate d'éthyle, lave à l'eau, sèche et élimine le solvant sous pression réduite à 40 °C. Après chromatographie sur silice (éluant méthanol-solution aqueuse d'acétate d'éthyle (0,05M) 95-5), on obtient 0,277 g de produit attendu.

METHODE B :

On chauffe 1 heure et demie à 50 °C sous atmosphère inerte 0,422 g du composé obtenu au stade B de l'exemple 18 dans 3 cm3 de tétrahydrofuranne en présence de 3 cm3 d'acide chlorhydrique 2N, refroidit à température ambiante, verse dans une solution aqueuse de bi carbonate de sodium, extrait à l'acétate d'éthyle, lave à l'eau, sèche et élimine le solvant sous pression réduite à 40 °C. On obtient 0,392 g de produit brut que l'on purifie par chromatographie sur silice (éluant : éthanol-solution aqueuse d'acétate d'ammonium (0,05M) 7-3).

$[\alpha]_D$ = +190° ± 3° (c = 1% $CHCl_3$)

| Analyse sur produit séché à 150 °C : $C_{34}H_{39}NO_3$ : 509,69 | | | | | | |
|---|---|---|---|---|---|---|
| Calculé<br>Trouvé | C% | 80,1<br>79,7 | H% | 7,7<br>7,8 | N% | 2,75<br>2,6 |

Exemple 20 : 11béta-[4-[2-(diméthylamino) éthoxy] phényl] 17alpha-(4-hydroxyphényl) estra-1 ,3,5 (10) trièn-3,17béta-diol.

On opère comme au stade A de l'exemple 2 en utilisant au départ 8,9 g de chlorure de cérium dans 40 cm3 de tétrahydrofuranne, 30 cm3 de solution de bromure de triméthyl silyloxy phényl magnésium 0,8N dans le tétrahydrofuranne et 2,36 g de 11béta-[4-[2-(diméthylamino) éthoxy] phényl] 3-hydroxy estra-1,3,5 (10)-trièn-17-one préparé comme indiqué dans le brevet européen 0 097 572. On obtient 8,25 g de produit dont la fonction phénol en 17 alpha est protégée par un radical triméthylsilyl. On dissout 3,3 g de ce produit dans 22 cm3 de méthanol, ajoute 4,35 g de silice, agite 3 heures à température ambiante, ajoute 1,8 cm3 de triéthylamine, filtre et élimine le solvant sous pression réduite à 40 °C. On obtient 2,638 g de produit attendu brut que l'on chromatographie sur silice (éluant : chlorure de méthylène-méthanol-ammoniaque 90-10-0,5) puis recristallise dans le méthanol. F = 308 °C.

$[\alpha]_D$ = -131° ± 2,5° (c = 0,25% éthanol).

| Analyse sur produit séché à 130 ° C : $C_{34}H_{41}NO_4$ = 527,31 | | | | | | |
|---|---|---|---|---|---|---|
| Calculé<br>Trouvé | C% | 77,38<br>77,4 | H% | 7,83<br>7,9 | N% | 2,65<br>2,6 |

Exemple 21 : 11béta-[4-[2-(diméthylamino) éthoxy] phényl] 17-(4-hydroxyphényl) estra-1 ,3,5 (10),16-tétraèn-3-ol.

On opère comme à l'exemple 2 stade B en utilisant 0,353 g du produit préparé à l'exemple 20 dans 7,5 cm3 de méthanol, 3 cm3 de tétrahydrofuranne et 7,85 cm3 d'acide chlorhydrique 2N. On obtient 0,356 g de produit brut que l'on purifie par chromatographie sur silice (éluant : méthanol-solution aqueuse d'acétate d'ammonium (0,05M) 9-1 puis chlorure de méthylène-méthanol-hydroxyde de sodium 90-10-0,5).
$[\alpha]_D$ = -135 ° ± 2,5 ° (c = 1% éthanol).

| Analyse sur produit séché à 150 ° C : $C_{34}H_{39}NO_3$ | | | | | | |
|---|---|---|---|---|---|---|
| Calculé<br>Trouvé | C% | 80,1<br>79,8 | H% | 7,7<br>7,7 | N% | 2,75<br>2,6 |

ETUDE PHARMACOLOGIQUE DES PRODUITS DE L'INVENTION

1. Activité anti-proliférative des produits sur la croissance de cellules tumorales mammaires MCF-7.

Description du test :

a) culture cellulaire :
Les lignées MCF-7 sont maintenues en culture en milieuSVF(1) à 37 C en atmosphère humide contenant 5% de $CO_2$. Les cellules à subconfluence sont récoltées par trypsination (trypsine 0,05%, EDTA 0,02 %) puis rincées par centrifugation douce. Un échantillon des cellules en suspension est compté sur cellule de Malassez.

b) étude de la croissance :

Les cellules resuspendues dans le milieu SVF sont ensemencées à raison de 30.000 cellules par puits dans des plaques multipuits (24 puits de 2,5 cm2) . Vingt-quatre heures après l'ensemencement (J0), le produit à tester est ajouté au milieu en solution éthanolique (concentration finale en éthanol : 0,1%) , à la concentration de $10^{-6}$M, les puits contrôles recevant la même concentration en éthanol. Les milieux sont renouvelés toutes les 48 heures. En fin d'expérience (J6), le milieu est asp ré et les cellules sont immédiatement fixées par 150 µl de méthanol afin de doser l'ADN. L'activité anti-proliférative des produits est évaluée par les capacité à inhiber l'augmentation d'ADN.

c) Dosage de l'ADN :

l'ADN est dosé par une méthode fluorimétrique utilisant le DABA (acide 3,5 diaminobenzoïque) (2) 150 µl de DABA est ajouté dans chaque puits ; les plaques sont alors incubées 45 mn à 56 ° C, puis 1,5 ml d'HCl 1N est ajouté. La fluorescence est mesurée à l'aide d'un fluorimètre ( longueur excitatrice : 400 nm, longueur d'onde d'émission : 500 nm).
La quantité d'ADN par puits est évaluée par rapport à une gamme étalon obtenue en traitant dans les mêmes conditions un standard d'ADN de thymus de veau.

Résultats :

La concentration en nM qui inhibe de 50% la croissance descellules $MCF_7$ (CI50) a été déterminée de la manière indiquée ci-dessus pour les produits des exemples 2, 3, 4, 5 et 6. On a obtenu les résultats suivants :

Produit de l'exemple 2 : CI 50 = 500 nM
Produit de l'exemple 3 : CI 50 = 800 nM
Produit de l'exemple 4 : CI 50 = 400 nM
Produit de l"exemple 5 : CI 50 = 1000 nM
Produit de l'exemple 6 : CI 50 = 50 nM
(produit A)
Produit de l'exemple 6 : CI 50 = 50 nM
(produit B)

(1) Le milieu de culture sérum de veau foetal (SVF) est préparé comme suit :

Milieu MEM (minimal Essential Medium) auquel sont ajoutés

- acides aminés non essentiels (GIBCO),
- péni-strepto (penicilline 100U/ml, streptomycine 0,1 mg/ml),
- fungizone 0,1%,
- insuline (50 ng/ml), - sérum de veau foetal (10% concentration finale) débarassé de stéroïdes endrogènes.

(2) Puzas et Goddman, Analytical Biochemistry, Vol .86, p. 50, (1978).

2) Etude de l'activité des produits de l'invention sur les récepteurs hormonaux :

Récepteur Progestogène de l'utérus de lapine :

Des lapines impubères d'environ 1 kg recoivent une application cutanée de 25 g d'estradiol, 5 jours après ce traitement, les animaux sont sacrifiés, les utérus sont prélevés, pesés et homogénéisés à 0 °C, à l'aide d'un Potter teflon-verre dans une solution tamponnée TS (Tri 10mM, saccharose 0,25 M, HCl pH 7,4) (1 g de tissu pour 50 ml de TS). L'homogénat est ensuite ultracentrifugé (105 000 g x 90 mn) à 0 °C. Des aliquotes du surnageant ainsi obtenu, sont incubées à 0 °C pendant un temps t, avec une concentration constante (T) de produit R tritié (17,21-diméthyl 19-nor 4,9- pregnadien-3,20-dione) en présence de concentrations croissantes (0 - 2500. $10^{-9}$M) soit de R froid, soit de progestérone froide, soit du produit froid à tester. La concentration de R tritié lié (B) est ensuite mesurée dans chaque incubat par la technique d'adsorption au charbon dextran.

Récepteur glucocorticoïde du thymus de rat :

Des rats mâles Sprague Dawley EOPS de 160 à 200 g sont surrénalectomisés. 4 à 8 jours après cette ablation, les animaux sont sacrifiés et les thymus sont prélevés et homogénéisés à 0 °C dans un tampon Tris 10mM, saccharose 0,25M, dithiothreitol 2mM, HCl pH 7,4 à l'aide d'un Potter polytétrafluoroéthylène-verre (1 g de tissu pour 10 ml de TS). L'homogénat est ensuite ultracentrifugé (105 000 g x 90 mn) à 0 °C. Des aliquotes du surnageant ainsi obtenu, sont incubées à 0° C pendant un temps (t) avec une concentration constante (T) de dexaméthasone tritiée en présence de concentrations croissantes (0 - 2500.$10^{-9}$M) soit de dexaméthasone froide, soit du produit froid à tester. La concentration de la dexaméthasone tritiée liée (B) est ensuite mesurée dans chaque incubat par la technique d'adsorption au charbon-dextran.

Calcul de l'affinité relative de liaison :

Le calcul de l'affinité relative de liaison (ARL) est identique pour tous les récepteurs.

On trace les 2 courbes suivantes : le pourcentage de l'hormone tritiée liée $\frac{B}{T}$ en fonction du logarithme de la concentration de l'hormone de référence froide et $\frac{B}{T}$ en fonction du logarithme de la concentration du produit froid testé.

On détermine la droite d'équation :

$$I_{50} = (\frac{B}{T}\text{ max.} + \frac{B}{T}\text{ min.})/2$$

| | |
|---|---|
| $\frac{B}{T}$ max. = | pourcentage de l'hormone tritiée liée pour une incubation de cette hormone tritiée à la concentration (T). |
| $\frac{B}{T}$ min. = | pourcentage de l'hormone tritiée liée pour une incubation de cette hormone tritiée à la concentration (T) en présence d'un grand excès d'hormone froide (2500.$10^{-9}$M). |

Les intersections de la droite $I_{50}$ et des courbes permettent d'évaluer les concentrations de l'hormone de référence froide (CH) et du produit froid testé (CX) qui inhibent de 50 % la liaison de l'hormone tritiée sur le récepteur.

L'affinité relative de liaison (ARL) du produit testé est déterminée par l'équation :

$$ARL = 100 \frac{(CH)}{(CX)}$$

Les résultats obtenus sont les suivants :

| Temps d'incubation à 0 ° C | Progestogène | | Glucocorticoïde | |
|---|---|---|---|---|
| Produits des exemples | 2H | 24H | 2H | 24H |
| 1 | 48 | 259 | 94 | 68 |
| 2 | 32 | 183 | 61 | 58 |
| 4 | 51 | 264 | 65 | 69 |
| 6 | 33 | 35 | 133 | 97 |
| 7 | 32 | 150 | 71 | 57 |
| 9 | 22 | 110 | 39 | 40 |
| 10 | 47 | 189 | 106 | 112 |
| 11 (produit A) | 38 | 230 | 56 | 45 |
| 11 (produit B) | 12 | 49 | 47 | 40 |
| 12 (produit A) | 24 | 131 | 90 | 76 |
| 13 (produit A) | 34 | 122 | 153 | 145 |
| 13 (produit B) | 1,9 | 1,7 | 76 | 63 |
| 14 | 5 | 67 | 98 | 129 |

Conclusion :

Les produits étudiés, particulièrement les produits des exemples 1 et 4 présentent une affini té très marquée pour les récepteurs glucocorticoïde et progestogène.

Des résultats obtenus, on peut conclure que les produits peuvent présenter des activités agonistes ou antagonistes, des glucocorticoïdes et des progestogènes.

**Revendications**

**Revendications pour les Etats contractants suivants : AT, BE, CH, DE, GB, IT, LI, LU, NL, SE**

**1.** Les produits de formule générale I :

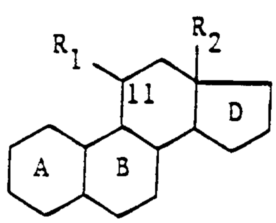

(I)

dans laquelle R1 représente

- un radical alkyle, linéaire ou ramifié, ayant de 1 à 12 atomes de carbone, ou un radical vinyle, isopropényle, allyle, 2-méthylallyle ou isobutényle, tous ces radicaux étant éventuellement substitués par :
  . les radicaux thiométhyle, thioéthyle,
  . les atomes d'halogènes,
  . le radical diméthylamino,
- un radical cycloalkyle ou cycloalkényle
- un radical phényle, benzyle ou un radical aryle hétérocyclique choisi parmi les radicaux thiényle, furyle, isothiényle, isofuryle, thiazolyle, isothiazolyle, oxazolyle, isoxazolyle, thiadiazolyle, pyridinyle ou pipéridinyle ces radicaux étant eux même éventuellement substitués par un ou plusieurs radicaux choisis parmi les radicaux :
  . alkyle ayant de 1 à 4 atomes de carbone
  . alkoxy ayant de 1 à 4 atomes de carbone
  . vinyloxy ou allyloxy
  . les atomes d'halogènes
  . trifluoromethyle,
  . alkylthio ayant de 1 à 4 atomes de carbone éventuellement oxydé sous forme de sulfoxyde ou de sulfone
  . acyle tel que acétyle, propionyle, butyryle ou benzoyle
  . un radical amino éventuellement substitué par un ou deux radicaux alkyle ayant de 1 à 8 atomes de carbone ou incorporé dans un hétérocycle comportant éventuellement un autre hétéroatome choisi dans le groupe formé par l'oxygène, l'azote et le soufre
  . un radical amino éventuellement substitué alkyle tel que amino methyle ou aminoéthyle, diméthylamino méthyle ou diméthylamino éthyle
  . un radical amino éventuellement substitué alkyloxy tel que diméthylamino éthyloxy
  . le radical triméthylsilyle
  . un radical hydroxy éventuellement acylé tel que acétoxy ou un radical de formule :

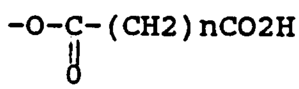

  dans lequel n = 2 à 5
  . un radical carboxy libre ou estérifié
  . un radical cyano
  . un radical aryle lui même éventuellement substitué par des radicaux alkyle, alkoxy, alkylthio, amino alkyle ou dialkyle amino tels que définis ci-dessus,
  étant entendu que l'atome immédiatement adjacent au carbone en 11 étant un atome de carbone, $R_2$ représente un radical méthyle ou éthyle, les cycles A et B ayant l'une des structures suivantes :

a) soit A et B représentent le groupement :

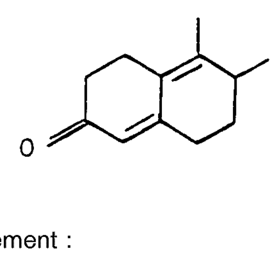

b) soit A et B représentent le groupement :

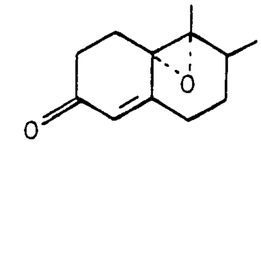

c) soit A et B représentent le groupement :

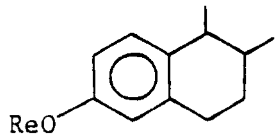

dans lequel Re représente un atome d'hydrogène, un radical alkyle ayant de 1 à 6 atomes de carbone éventuellement substitué ou un radical acyle,

le cycle D ayant l'une des structures suivantes :

a) Soit D représente le groupement :

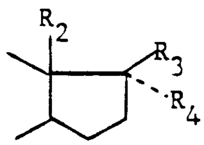

dans lequel l'un des substituants $R_3$ ou $R_4$ représente un radical hydroxyle éventuellement protégé ou acylé ou un radical alcoxyle et l'autre des substituants $R_3$ ou $R_4$ représente un radical aryle éventuellement substitué,

b) Soit D représente le groupement :

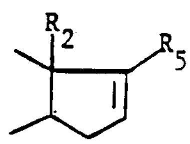

dans lequel $R_5$ représente un radical aryle éventuellement substitué ainsi que les sels d'addition des produits de formule I avec les acides et les bases et à l'exception du produit de formule I dans laquelle les cycles A et B représentent le groupement :

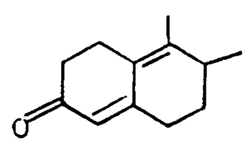

$R_1$ représente le radical :

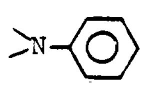

$R_2$ représente un radical méthyle, $R_3$ représente un radical hydroxyle et $R_4$ représente un radical phényle.

**2.** Les produits de formule générale I telle que définie à la revendication 1 dans laquelle $R_1$ représente un radical aryle éventuellement substitué.

**3.** Les produits de formule générale I telle que définie à la revendication 1 ou 2 dans laquelle A et B représentent le groupement :

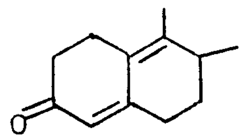

4. Les produits de formule générale I telle que définie à l'une des revendications 1 à 3 dans laquelle $R_3$ représente un radical hydroxyle ou méthoxy et $R_4$ représente un radical aryle éventuellement substitué.

5. Les produits de formule générale I telle que définie à l'une des revendications 1 à 4, répondant aux formules suivantes :
   - la 11béta,17alpha-bis/4-(diméthylamino) phényl/17béta-hydroxy estra-4,9-dièn-3-one,
   - la 11béta-/4-(diméthylamino) phényl/ 17béta-hydroxy 17alpha-(3-méthoxy phényl) estra-4,9-dièn-3-one.

6. Procédé de préparation des produits de formule générale I tels que définis à la revendication 1 caractérisé en ce que :
   a) soit l'on soumet un produit de formule II :

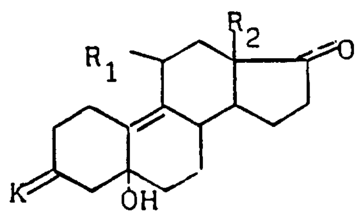

II

dans laquelle $R_1$ et $R_2$ ont la signification indiquée à la revendication 1 et K représente un groupe cétonique protégé,

i) d'abord à l'action d'un réactif organométallique dérivé d'un radical aryle éventuellement substitué que peut représenter $R_3$ ou $R_4$

ii) puis éventuellement ou bien à une séparation des isomères obtenus ou bien à une réaction de déshydration en position 16(17)

iii) puis éventuellement, dans un ordre quelconque, à l'action d'un agent de protection, d'alkylation ou d'acylation du radical hydroxy que représente $R_3$ ou $R_4$ et nécessairement à l'action d'un agent de déshydratation susceptible de libérer la fonction cétone, pour obtenir les produits de formules IA

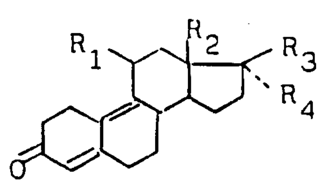

IA

et I'A

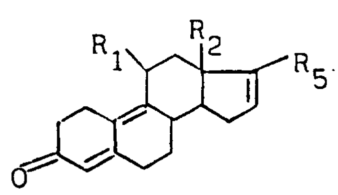

I'A

formules dans lesquelles $R_1$, $R_2$, $R_3$, $R_4$ et $R_5$ ont la signification indiquée à la revendication 1 et produits de formules IA ou I'A que si désiré l'on traite ou bien par un réactif d'oxydation, pour obtenir respectivement un produit de formule IB :

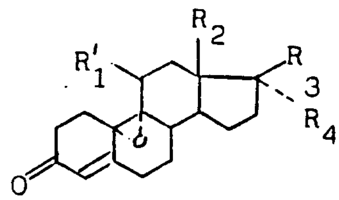

IB

et I'B :

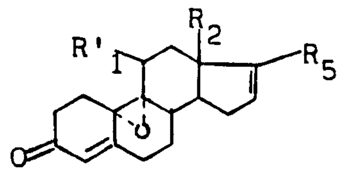

I'B

formules IB et I'B dans lesquelles $R_2$, $R_3$, $R_4$ et $R_5$ ont la signification indiquée à la revendication et $R'_1$ a la signification indiquée à la revendication 1 pour $R_1$ étant entendu toutefois que $R'_1$ comporte un atome d'azote oxydé si $R_1$ comporte un atome d'azote, produits de formules $I_B$ et $I'_B$ dans lesquelles le radical $R'_1$ comporte un atome d'azote oxydé que si désiré l'on traite par un agent de réduction pour obtenir un produit de formule $I_B$ ou $I'_B$ dans laquelle $R'_1$ comporte un atome d'azote non oxydé,

ou bien l'on traite les produits de formules IA et I'A par un agent d'aromatisation puis éventuellement par un réactif d'alkylation ou d'acylation pour obtenir respectivement les produits de formule IC :

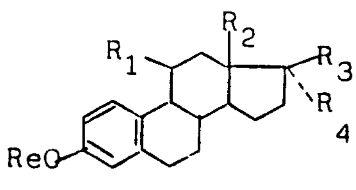

IC

et I'C

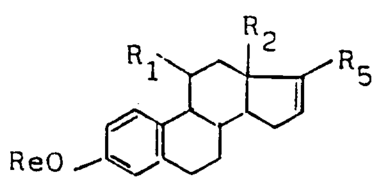

I'C

formules IC et I'C dans lesquelle Re, $R_1$, $R_2$, $R_3$, $R_4$ et $R_5$ ont la signification indiquée à la revendication 1,

b) soit l'on soumet un produit de formule III :

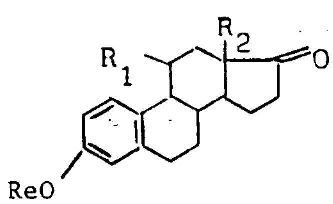

III

dans laquelle $R_1$ et $R_2$ ont la signification indiquée à la revendication 1, à l'action d'un réactif organométallique dérivé d'un radical aryle éventuellement substitué que peut représenter $R_3$ ou $R_4$

puis éventuellement ou bien dans un ordre quelconque à une séparation des isomères obtenus et à l'action éventuelle d'un agent de protection, d'alkylation ou d'acylation du radical hydroxy que représente $R_3$ ou $R_4$ ou bien à une réaction de déshydratation en position 16(17) et que si désiré l'on soumet les produits de formules IA, I'A, IB, I'B, IC et I'C à l'action d'une base ou d'un acide pour obtenir les sels correspondants.

**7.** A titre de médicaments, les produits de formule générale I telle que définie à la revendication 1.

**8.** A titre de médicaments, les produits de formule générale I telle que définie à l'une des revendications 2 à 5.

**9.** Les compositions pharmaceutiques comprenant, comme principe actif, au moins un médicament défini à l'une quelconque des revendications 7 ou 8.

**10.** A titre de produits industriels nouveaux, les produits répondant aux formules suivantes :
3,3-(1,2 éthanediyl) acétal cyclique de 5 alpha-hydroxy 11 Béta-(4-méthoxy phényl) estr-9-en-3,17-dione
3,3-(1,2-éthanediyl) acétal cyclique de 5 alpha-hydroxy 11 béta-/4-(méthylthio) phényl/ estr-9-en-3,17-dione
11 béta-/4-(diméthylamino) phényl/estra-4,9-diène 3,17-dione.
11 béta-/4-(diméthylamino) phényl/ 3-hydroxy estra 1,3,5 (10)-trièn-17-one.

**Revendications pour l'Etat contractant suivant : ES**

**1.** Procédé pour préparer les produits de formule générale I :

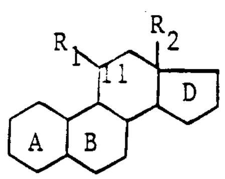

dans laquelle R1 représente
- un radical alkyle, linéaire ou ramifié, ayant de 1 à 12 atomes de carbone, ou un radical vinyle, isopropényle, allyle, 2-méthylallyle ou isobutényle, tous ces radicaux étant éventuellement substitués par :
  . les radicaux thiométhyle, thioéthyle,
  . les atomes d'halogènes,
  . le radical diméthylamino,
- les radicaux cycloalkyle ou cycloalkényle
- les radicaux phényle, benzyle ou un radical aryle hétérocyclique choisi parmi les radicaux thiényle, furyle, isothiényle, isofuryle, thiazolyle, isothiazolyle, oxazolyle, isoxazolyle, thiadiazolyle, pyridinyle ou pipéridinyle ces radicaux étant eux même éventuellement substitués par un ou plusieurs radicaux choisis parmi les radicaux :
  . alkyle ayant de 1 à 4 atomes de carbone
  . alkoxy ayant de 1 à 4 atomes de carbone
  . vinyloxy ou allyloxy
  . les atomes d'halogènes
  . trifluorométhyle,
  . alkylthio ayant de 1 à 4 atomes de carbone éventuellement oxydé sous forme de sulfoxyde ou de sulfone
  . acyle tel que acétyle, propionyle, butyryle ou benzoyle
  . un radical amino éventuellement substitué par un ou deux radicaux alkyle ayant de 1 à 8 atomes de carbone ou incorporé dans un hétérocycle comportant éventuellement un autre hétéroatome choisi dans le groupe formé par l'oxygène, l'azote et le soufre
  . un radical amino éventuellement substitué alkyle tel que amino méthyle ou aminoéthyle, diméthylamino méthyle ou diméthylamino ethyle

. un radical amino éventuellement substitué alkyloxy tel que diméthylamino éthyloxy
. le radical triméthylsilyle
. un radical hydroxy éventuellement acylé tel que acétoxy ou un radical de formule :

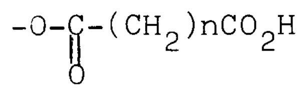

dans lequel n = 2 à 5
. un radical carboxy libre ou estérifié
. un radical cyano
. un radical aryle lui-même éventuellement substitué par des radicaux alkyle, alkoxy, alkylthio, amino alkyle ou dialkyle amino tels que définis ci-dessus, étant entendu que, l'atome immédiatement adjacent au carbone en 11 étant un atome de carbone, $R_2$ représente un radical méthyle ou éthyle, les cycles A et B ayant l'une des structures suivantes :

a) soit A et B représentent le groupement :

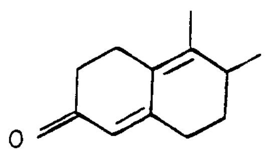

b) soit A et B représentent le groupement :

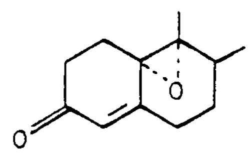

c) soit A et B représentent le groupement :

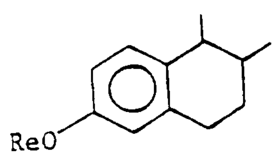

dans lequel Re représente un atome d'hydrogène, un radical alkyle ayant de 1 à 6 atomes de carbone éventuellement substitué ou un radical acyle,

le cycle D ayant l'une des structures suivantes :

a) Soit D représente le groupement :

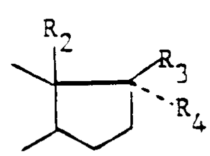

dans lequel l'un des substituants $R_3$ ou $R_4$ représente un radical hydroxyle éventuellement protégé ou acylé ou un radical alcoxyle et l'autre des substituants $R_3$ ou $R_4$ représente un radical aryle éventuellement substitué,

b) Soit D représente le groupement :

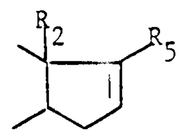

dans lequel $R_5$ représente un radical aryle éventuellement substitué ainsi que les sels d'addition des produits de formule I avec les acides et les bases et à l'exception du produit de formule I dans laquelle les cycles A et B représentent le groupement :

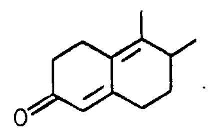

$R_1$ représente le radical :

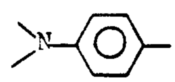

$R_2$ représente un radical méthyle, $R_3$ représente un radical hydroxyle et $R_4$ représente un radical phényle, caractérisé en ce que :

a) soit l'on soumet un produit de formule II :

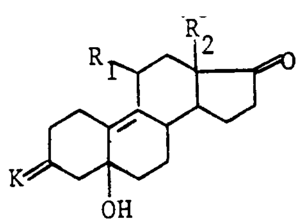

II

dans laquelle $R_1$ et $R_2$ ont la signification indiquée précédemment et K représente un groupe cétonique protégé,

i) d'abord à l'action d'un réactif organométallique dérivé d'un radical aryle éventuel lement substitué que peut représenter $R_3$ ou $R_4$

ii) puis éventuellement ou bien à une séparation des isomères obtenus ou bien à une réaction de déshydration en position 16(17)

iii) puis éventuellement, dans un ordre quelconque, à l'action d'un agent de protection, d'alkylation ou d'acylation du radical hydroxy que représente $R_3$ ou $R_4$ et nécessairement à l'action d'un agent de déshydratation susceptible de libérer la fonction cétone, pour obtenir les produits de formules IA

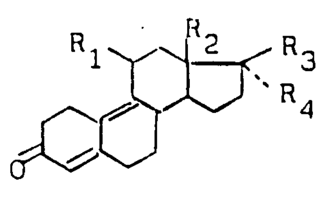

IA

et l'A

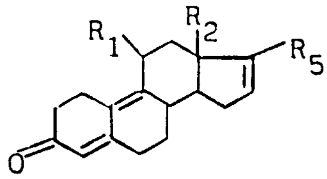

I'A

formules dans lesquelles $R_1$, $R_2$, $R_3$, $R_4$ et $R_5$ ont la signification indiquée précédemment et produits de formules IA ou I'A que si désiré l'on traite ou bien par un réactif d'oxydation, pour obtenir respectivement un produit de formule IB :

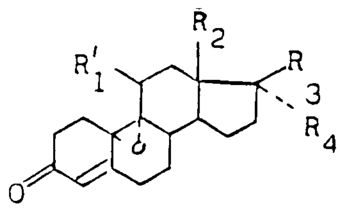

IB

et I'B :

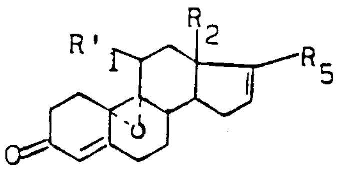

I'B

formules IB et I'B dans lesquelles $R_2$, $R_3$, $R_4$ et $R_5$ ont la signification indiquée précédemment et $R'_1$ a la signification indiquée précédemment pour $R_1$ étant entendu toutefois que $R'_1$ comporte un atome d'azote oxydé si $R_1$ comporte un atome d'azote, produits de formules $I_B$ et $I'_B$ dans lesquelles le -adical $R'_1$ comporte un atome d'azote oxydé que si désiré l'on traite par un agent de réduction pour obtenir un produit de formule $I_B$ ou $I'_B$ dans laquelle $R'_1$ comporte un atome d'azote non oxydé,

ou bien l'on traite les produits de formules IA et I'A par un agent d'aromatisation puis éventuellement par un réactif d'alkylation ou d'acylation pour obtenir respectivement les produits de formule IC :

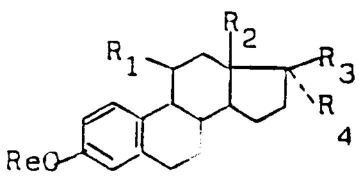

IC

et I'C

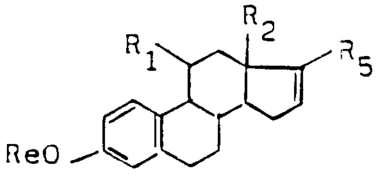

I'C

formules IC et I'C dans lesquelle Re, $R_1$, $R_2$, $R_3$, $R_4$ et $R_5$ ont la signification indiquée précédemment,

b) soit l'on soumet un produit de formule III :

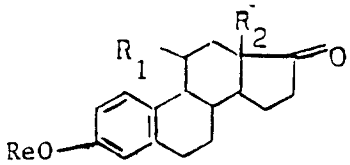

III

dans laquelle $R_1$ et $R_2$ ont la signification indiquée précédemment à l'action d'un reactif organométallique dérivé d'un radical aryle éventuellement substitué que peut représenter $R_3$ ou $R_4$ puis éventuellement ou bien dans un ordre quelconque à une séparation des isomères obtenus et à l'action éventuelle d'un agent de protection, d'alkylation ou d'acylation du radical hydroxy que représente $R_3$ ou $R_4$ ou bien à une réaction de déshydratation en position 16(17) et que si désiré l'on soumet les produits de formules IA, I'A, IB, I'B, IC et I'C à l'action d'une base ou d'un acide pour obtenir les sels correspondants.

**2.** Procédé selon la revendication 1, caractérisé en ce que l'on utilise au départ, un composé de formule (II) ou (III), dans laquelle $R_1$ represente un radical phényle éventuellement substitué.

**3.** Procédé selon la revedication 1 ou 2, caractérisé en ce que l'on prépare des produits de formule générale (I) dans laquelle A et B représentent le groupement :

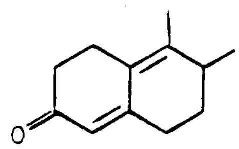

**4.** Procédé selon l'une quelconque des revendications 1 à 3, caractérisé en ce que l'on prépare des produits de formule générale (I) dans laquelle $R_3$ représente un radical hydroxyle ou méthoxy et $R_4$ représente un radical phényle éventuellement substitué.

**5.** Procédé selon l'une quelconque des revendications 1 à 4, caractérisé en ce que l'on prépare l'un quelconque des produits dont les noms suivent :
- la 11béta,17alpha-bis/4-(diméthylamino) phényl/17béta-hydroxy estra-4,9-dièn-3-one,
- la 11béta-/4-(diméthylamino) phényl/ 17béta-hydroxy 17alpha-(3-methoxy pnényl) estra-4,9-dièn-3-one.

**Revendications pour l'Etat contractant suivant : GR**

**1.** Procédé pour préparer les produits de formule générale I :

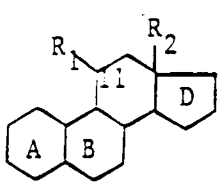

dans laquelle R1 représente
- un radical alkyle, linéaire ou ramifié, ayant de 1 à 12 atomes de carbone, ou un radical vinyle, isopropenyle, allyle, 2-méthylallyle ou isobuténylе, tous ces radicaux étant éventuellement substitués par :
  . les radicaux thiométhyle, thioéthyle,
  . les atomes d'halogènes,
  . le radical diméthylamino,
- les radicaux cycloalkyle ou cycloalkényle

- les radicaux phényle, benzyle ou un radical aryle hétérocyclique choisi parmi les radicaux thiényle, furyle, isothiényle, isofuryle, thiazolyle, isothiaxolyle, oxasolyle, isoxazolyle, thiadiazolyle, pyridinyle ou pipéridinyle ces radicaux étant eux même éventuellement substitués par un ou plusieurs radicaux choisis parmi les radicaux :
  . alkyle ayant de 1 à 4 atomes de carbone
  . alkoxy ayant de 1 à 4 atomes de carbone
  . vinyloxy ou allyloxy
  . les atomes d'halogènes
  . trifluorométhyle,
  . alkylthio ayant de 1 à 4 atomes de carbone éventuellement oxydé sous forme de sulfoxyde ou de sulfone
  . acyle tel que acétyle, propionyle, butyryle ou benzoyle
  . un radical amino éventuellement substitué par un ou deux radicaux alkyle ayant de 1 à 8 atomes de carbone ou incorporé dans un héterocycle comportant éventuellement un autre hétéroatome choisi dans le groupe formé par l'oxygène, l'azote et le soufre
  . un radical amino éventuellement substitué alkyle tel que amino méthyle ou aminoéthyle, diméthylamino méthyle ou diméthyiamino éthyle
  . un radical amino éventuellement substitué alkyloxy tel que diméthylamino éthyloxy
  . le radical triméthylsilyle
  . un radical hydroxy éventuellement acylé tel que acétoxy ou un radical de formule :

$$-O-\underset{\underset{O}{\|}}{C}-(CH_2)nCO_2H$$

  dans lequel n = 2 à 5
  . un radical carboxy libre ou estérifié
  . un radical cyano
  . un radical aryle lui-même éventuellement substitué par des radicaux alkyle, alkoxy, alkylthio, amino alkyle ou dialkyle amino tels que définis ci-dessus, étant entendu que, l'atome immédiatement adjacent au carbone en 11 étant un atome de carbone, $R_2$ représente un radical méthyle ou éthyle, les cycles A et B avant l'une des structures suivantes :

a) soit A et B représentent le groupement :

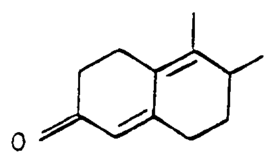

b) soit A et B représentent le groupement :

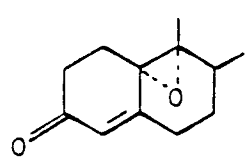

c) soit A et B représentent le groupement :

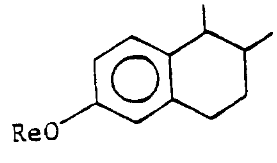

dans lequel Re représente un atome d'hydrogène, un radical alkyle ayant de 1 à 6 atomes de carbone éventuellement substitué ou un radical acyle,
le cycle D ayant l'une des structures suivantes :
a) Soit D représente le groupement :

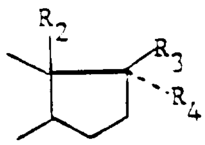

dans lequel l'un des substituants $R_3$ ou $R_4$ représente un radical hydroxyle éventuellement protégé ou acylé ou un radical alcoxyle et l'autre des substituants $R_3$ ou $R_4$ représente un radical aryle éventuellement substitué,
b) Soit D représente le groupement :

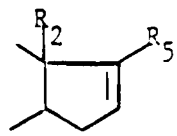

dans lequel $R_5$ représente un radical aryle éventuellement substitué ainsi que les sels d'addition des produits de formule I avec les acides et les bases et à l'exception du produit de formule I dans laquelle les cycles A et B représentent le groupement :

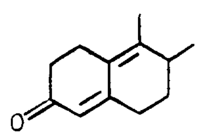

$R_1$ représente le radical :

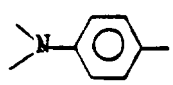

$R_2$ représente un radical méthyle, $R_3$ représente un radical hydroxyle et $R_4$ représente un radical phényle, caractérisé en ce que :
a) soit l'on soumet un produit de formule II :

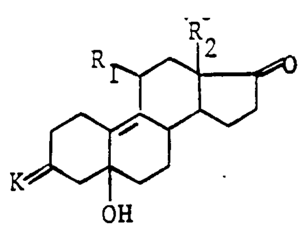

II

dans laquelle $R_1$ et $R_2$ ont la signification indiquée précédemment et K représente un groupe cétonique protégé,

i) d'abord à l'action d'un réactif organométallique dérivé d'un radical aryle éventuellement substitué que peut représenter $R_3$ ou $R_4$

ii) puis éventuellement <u>ou bien</u> à une séparation des isomères obtenus <u>ou bien</u> à une réaction de déshydration en position 16(17)

iii) puis éventuellement, dans un ordre quelconque, à l'action d'un agent de protection, d'alkylation ou d'acylation du radical hydroxy que représente $R_3$ ou $R_4$ et nécessairement à l'action d'un

agent de déshydratation susceptible de libérer la fonction cétone, pour obtenir les produits de formules IA

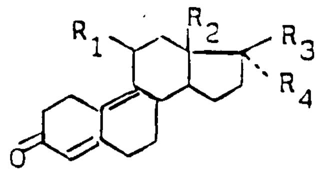

IA

et I'A

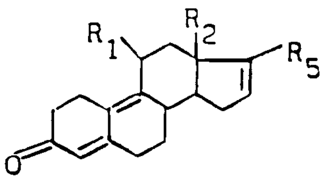

I'A

formules dans lesquelles $R_1$, $R_2$, $R_3$, $R_4$ et $R_5$ ont la signification indiquée précédemment et produits de formules IA ou I'A que si désiré l'on traite ou bien par un réactif d'oxydation, pour obtenir respectivement un produit de formule IB :

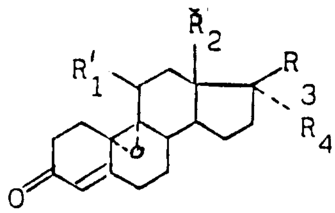

IB

et I'B :

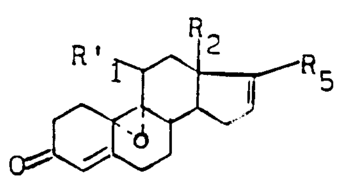

I'B

formules IB et I'B dans lesquelles $R_2$, $R_3$, $R_4$ et $R_5$ ont la signification indiquée précédemment et R'1 a la signification indiquée précédemment pour $R_1$ étant entendu toutefois que $R'_1$ comporte un atome d'azote oxydé si $R_1$ comporte un atome d'azote, produits de formules IB et I'B dans lesquelles le radical $R'_1$ comporte un atome d'azote oxydé que si désiré l'on traite par un agent de réduction pour obtenir un produit de formule IB ou I'B dans laquelle $R'_1$ comporte un atome d'azote non oxydé,
ou bien l'on traite les produits de formules IA et I'A par un agent d'aromatisation puis éventuellement par un réactif d'alkylation ou d'acylation pour obtenir respectivement les produits de formule IC :

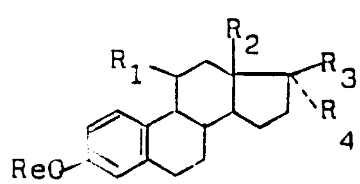

IC

et I'C

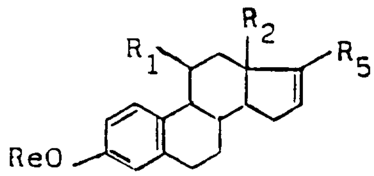

I'C

formules IC et I'C dans lesquelle Re, $R_1$, $R_2$, $R_3$, $R_4$ et $R_5$ ont la signification indiquée précédemment,

b) soit l'on soumet un produit de formule III :

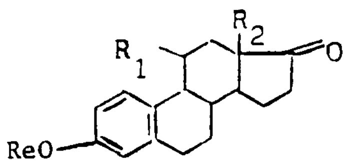

III

dans laquelle $R_1$ et $R_2$ ont la signification indiquée précèdem-ment à l'action d'un réactif organométallique dérivé d'un radical aryle éventuellement substitué que peut représenter $R_3$ ou $R_4$ puis éventuellement ou bien dans un ordre quelconque à une séparation des isomères obtenus et à l'action éventuelle d'un agent de protection, d'alkylation ou d'acylation du radical hydroxy que représente $R_3$ ou $R_4$ ou bien à une réaction de déshydratation en position 16(17) et que si désiré l'on soumet les produits de formules IA, I'A, IB, I'B, IC et I'C à l'action d'une base ou d'un acide pour obtenir les sels correspondants.

2. Procédé selon la revendication 1, caractérisé en ce que l'on utilise au départ, un composé de formule (II) ou (III), dans laquelle $R_1$ représente un radical phényle éventuellement substitué.

3. Procédé selon la revedication 1 ou 2, caractérisé en ce que l'on prépare des produits de formule générale (I) dans laquelle A et B représentent le groupement :

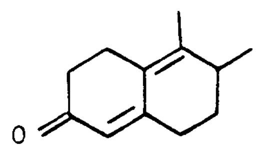

4. Procédé selon l'une quelconque des revendications 1 à 3, caractérisé en ce que l'on prépare des produits de formule générale (I) dans laquelle $R_3$ représente un radical hydroxyle ou méthoxy et $R_4$ représente un radical phényle éventuellement substitué.

5. Procédé selon l'une quelconque des revendications 1 à 4, caractérisé en ce que l'on prépare l'un quelconque des produits dont les noms suivent :
   - la 11béta,17alpha-bis/4-(diméthylamino) phényl/17béta-hydroxy estra-4,9-dièn-3-one,
   - la 11béta-/4-(diméthylamino) phényl/ 17béta-hydroxy 17 alpha-(3-méthoxy phényl) estra-4,9-dièn-3-one.

6. Procedé de préparation de compositions pharmaceutiques caractérisé en ce que l'on met, à titre de principe actif, l'un au moins des produits de formule générale (I), telle que définie à la revendication 1, ou l'un au moins de leurs sels d'addition avec les acides et les bases pharmaceutiquement acceptables, sous une forme destinée à cet usage.

7. Procédé de préparation de compositions pharmaceutiques caractérisé en ce que l'on met, à titre de principe actif, l'un au moins des produits de formule générale (I), telle que définie à l'une quelconque

des revendications 2 à 4, ou l'un au moins de leurs sels d'addition avec les acides et les bases pharmaceutiquement acceptables, sous une forme destinée à cet usage.

**8.** Procédé de préparation de compositions pharmaceutiques caractérisé en ce que l'on met, à titre de principe actif, l'un au moins des produits de formule générale (I), telle que définie à la revendication 1, dont les noms suivent :
- la 11béta,17alpha-bis/4-(diméthylamino) phényl/17béta-hydroxy estra-4,9-dièn-3-one,
- la 11béta-/4-(diméthylamino) phényl/ 17béta-hydroxy 17alpha-(3-méthoxy phényl) estra-4,9-dièn-3-one,

sous une forme destinée à cet usage.

**Claims**

**Claims for the following Contracting States : AT, BE, CH, DE, GB, IT, LI, LU, NL, SE**

**1.** The products of general formula I:

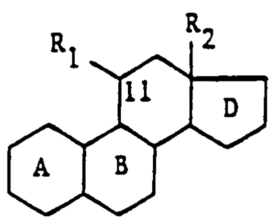

(I)

in which $R_1$ represents
- a linear or branched alkyl radical having 1 to 12 carbon atoms, or a vinyl, isopropenyl, allyl, 2-methylallyl or isobutenyl radical, all these radicals being optionally substituted by:
  . thiomethyl, thioethyl radicals,
  . halogen atoms,
  . the dimethylamino radical,
- a cycloalkyl or cycloalkenyl radical,
- a phenyl, benzyl radical or a heterocyclic aryl radical chosen from the following radicals: thienyl, furyl, isothienyl, isofuryl, thiazolyl, isothiazolyl, oxazolyl, isoxazolyl, thiadiazolyl, pyridinyl or piperidinyl, these radicals themselves being optionally substituted by one or more radicals chosen from the following radicals:
  . alkyl having 1 to 4 carbon atoms
  . alkoxy having 1 to 4 carbon atoms
  . vinyloxy or allyloxy
  . halogen atoms
  . trifluoromethyl,
  . alkylthio having 1 to 4 carbon atomsoptionally oxidized in the form of the sulphoxide or the sulphone
  . acyl such as acetyl, propionyl, butyryl or benzoyl
  . an amino radical optionally substituted by one or two alkyl radicals having 1 to 8 carbon atoms or incorporated in a heterocycle optionally containing another heteroatom chosen from the group formed by oxygen, nitrogen and sulphur
  . an optionally substituted amino alkyl radical such as amino methyl or aminoethyl, dimethylamino methyl or dimethylamino ethyl
  . an optionally substituted amino alkyloxy radical such as dimethylamino ethyloxy
  . the trimethylsilyl radical
  . an optionally acylated hydroxy radical such as acetoxy or a radical of formula:

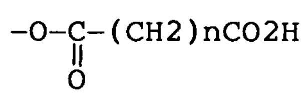

in which n = 2 to 5

. a free or esterified carboxy radical

. a cyano radical

. an aryl radical itself optionally substituted by alkyl, alkoxy, alkylthio, amino alkyl or dialkyl amino radicals as defined above,

it being understood that when the atom immediately adjacent to the carbon in position 11 is a carbon atom, $R_2$ represents a methyl or ethyl radical, rings A and B having one of the following structures:

a) either A and B represent the group:

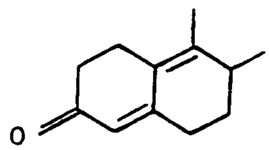

b) or A and B represent the group:

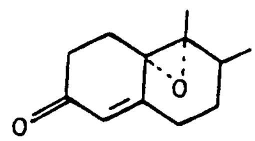

c) or A and B represent the group:

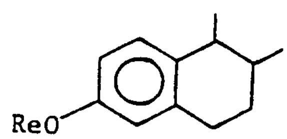

in which Re represents a hydrogen atom, an optionally substituted alkyl radical having 1 to 6 carbon atoms or an acyl radical,

ring D having one of the following structures:

a) either D represents the group:

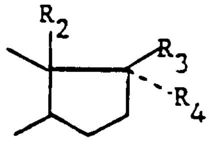

in which one of the substituents $R_3$ or $R_4$ represents an optionally protected or acylated hydroxyl radical or an alkoxyl radical and the other substituent $R_3$ or $R_4$ represents an optionally substituted aryl radical,

b) or D represents the group:

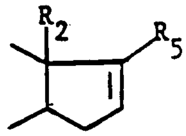

in which $R_5$ represents an optionally substituted aryl radical, as well as the addition salts of the products of formula I with the acids and bases and with the exception of the product of formula I in which rings A and B represent the group:

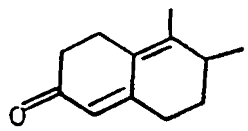

$R_1$ represents the radical:

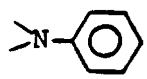

$R_2$ represents a methyl radical, $R_3$ represents a hydroxyl radical and $R_4$ represents a phenyl radical.

**2.** The products of general formula I as defined in claim 1 in which $R_1$ represents an optionally substituted aryl radical.

**3.** The products of general formula I as defined in claim 1 or 2 in which A and B represent the group:

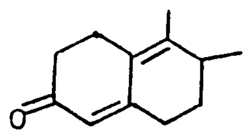

**4.** The products of general formula I as defined in one of claims 1 to 3 in which $R_3$ represents a hydroxyl or methoxy radical and $R_4$ represents an optionally substituted aryl radical.

**5.** The products of general formula I as defined in one of claims 1 to 4, corresponding to the following formulae:
- 11beta,17alpha-bis/4-(dimethylamino) phenyl/17beta-hydroxy estra-4, 9-dien-3-one,
- 11beta-/4-(dimethylamino) phenyl/ 17beta-hydroxy 17alpha-(3-methoxy phenyl) estra-4,9-dien-3-one.

**6.** Preparation process for the products of general formula I as defined in claim 1 characterized in that:
a) either a product of formula II:

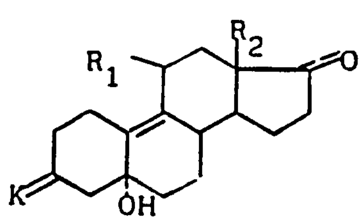

II

in which $R_1$ and $R_2$ have the meaning indicated in claim 1 and K represents a protected ketone group, is subjected
i) first, to the action of an organometallic reagent derived from an optionally substituted aryl radical that can be represented by $R_3$ or $R_4$

ii) then optionally either to a separation of the isomers obtained or to a dehydration reaction in position 16(17)
iii) then optionally, in any order, to the action of a protection, alkylation or acylation agent of the hydroxy radical that is represented by $R_3$ or $R_4$ and necessarily to the action of a dehydration agent capable of releasing the ketone function, in order to obtain the products of formula IA:

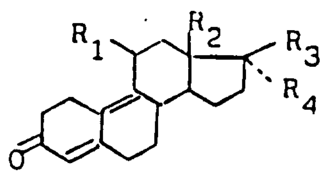

IA

and I'A

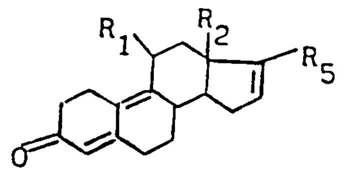

I'A

in which formulae $R_1$, $R_2$, $R_3$, $R_4$ and $R_5$ have the meaning indicated in claim 1 and which products of formula IA or I'A, if desired, are treated either by an oxidation reagent, in order to obtain respectively a product of formula IB:

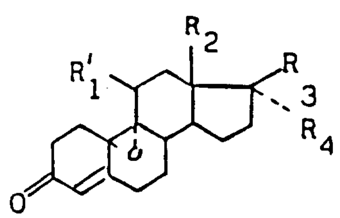

IB

and I'B:

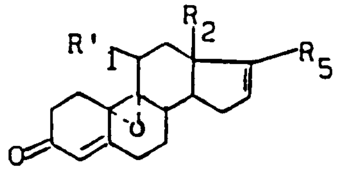

I'B

in which formulae IB and I'B $R_2$, $R_3$, $R_4$ and $R_5$ have the meaning indicated in claim 1 and $R'_1$ has the meaning indicated in claim 1 for $R_1$, it being understood however that $R'_1$ contains an oxidized nitrogen atom if $R_1$ contains a nitrogen atom, in which products of formula IB and I'B the $R'_1$ radical contains an oxidized nitrogen atom which if desired is treated by a reducing agent in order to obtain a product of formula IB or I'B in which $R'_1$ contains a non-oxidized nitrogen atom, or the products of formulae IA and I'A are treated by an aromatization agent then optionally by an

alkylation or acylation reagent in order to obtain respectively the products of formula IC:

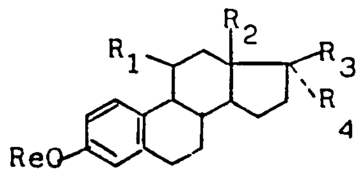

IC

and I'C

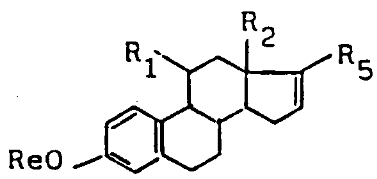

I'C

in which formulae IC and I'C Re, $R_1$, $R_2$, $R_3$, $R_4$ and $R_5$ have the meaning indicated in claim 1,
b) or a product of formula III:

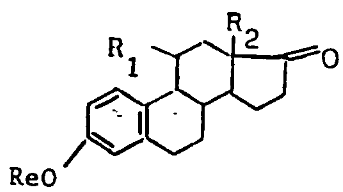

III

in which $R_1$ and $R_2$ have the meaning indicated in claim 1, is subjected to the action of an organometallic reagent derived from an optionally substituted aryl radical that can be represented by $R_3$ or $R_4$ then optionally either in any order to a separation of the isomers obtained and to the optional action of a protection, alkylation or acylation agent of the hydroxy radical that is represented by $R_3$ or $R_4$ or to a dehydration reaction in position 16(17) and that if desired the products of formulae IA, I'A, IB, I'B, IC and I'C are subjected to the action of a base or an acid in order to obtain the corresponding salts.

**7.** As medicaments, the products of general formula I as defined in claim 1.

**8.** As medicaments, the products of general formula I as defined in one of claims 2 to 5.

**9.** The pharmaceutical compositions containing, as active ingredient, at least one medicament defined in any one of claims 7 or 8.

**10.** As new industrial products, the products corresponding to the following formulae:
5 alpha-hydroxy 11 beta-(4-methoxy phenyl) estr-9-en-3,17-dione cyclic 3,3-(1,2 ethanediyl) acetal
5 alpha-hydroxy 11 beta-/4-(methylthio) phenyl/ estr-9-en-3,17-dione cyclic 3,3-(1,2-ethanediyl) acetal
11 beta-/4-(dimethylamino) phenyl/estra-4,9-diene 3,17-dione
11 beta-/4-(dimethylamino) phenyl/3-hydroxy estra 1,3,5(10)-trien-17-one.

**Claims for the following Contracting State : ES**

**1.** Preparation process for the products of general formula I:

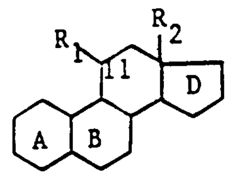

in which $R_1$ represents

- a linear or branched alkyl radical having 1 to 12 carbon atoms, or a vinyl, isopropenyl, allyl, 2-methylallyl or isobutenyl radical, all these radicals being optionally substituted by:
  . thiomethyl, thioethyl radicals,
  . halogen atoms,
  . the dimethylamino radical,
- a cycloalkyl or cycloalkenyl radical,
- a phenyl, benzyl radical or a heterocyclic aryl radical chosen from the following radicals: thlenyl, furyl, isothienyl, isofuryl, thiazolyl, isothiazolyl, oxazolyl, isoxazolyl, thiadiazolyl, pyridinyl or piperidinyl, these radicals themselves being optionally substituted by one or more radicals chosen from the following radicals:
  . alkyl having 1 to 4 carbon atoms
  . alkoxy having 1 to 4 carbon atoms
  . vinyloxy or allyloxy
  . halogen atoms
  . trifluoromethyl,
  . alkylthio having 1 to 4 carbon atoms optionally oxidized in the form of the sulphoxide or the sulphone
  . acyl such as acetyl, propionyl, butyryl or benzoyl
  . an amino radical optionally substituted by one or two alkyl radicals having 1 to 8 carbon atoms or incorporated in a heterocycle optionally containing another heteroatom chosen from the group formed by oxygen, nitrogen and sulphur
  . an optionally substituted amino alkyl radical such as amino methyl or aminoethyl, dimethylamino methyl or dimethylamino ethyl
  . an optionally substituted amino alkyloxy radical such as dimethylamino ethyloxy
  . the trimethylsilyl radical
  . an optionally acylated hydroxy radical such as acetoxy or a radical of formula:

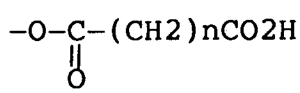

  in which n = 2 to 5
  . a free or esterified carboxy radical
  . a cyano radical
  . an aryl radical itself optionally substituted by alkyl, alkoxy, alkylthio, amino alkyl or dialkyl amino radicals as defined above,

it being understood that when the atom immediately adjacent to the carbon in position 11 is a carbon atom, $R_2$ represents a methyl or ethyl radical, rings A and B having one of the following structures:

a) either A and B represent the group:

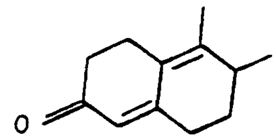

b) or A and B represent the group:

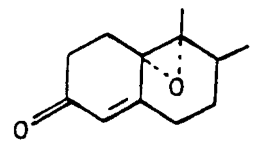

c) or A and B represent the group:

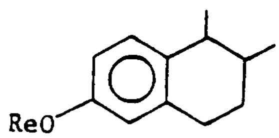

in which Re represents a hydrogen atom, an optionally substituted alkyl radical having 1 to 6 carbon atoms or an acyl radical,

ring D having one of the following structures:

a) either D represents the group:

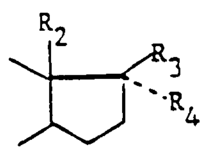

in which one of the substituents $R_3$ or $R_4$ represents an optionally protected or acylated hydroxyl radical or an alkoxyl radical and the other substituent $R_3$ or $R_4$ represents an optionally substituted aryl radical,

b) or D represents the group:

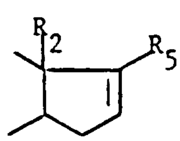

in which $R_5$ represents an optionally substituted aryl radical, as well as the addition salts of the products of formula I with the acids and bases and with the exception of the product of formula I in which rings A and B represent the group:

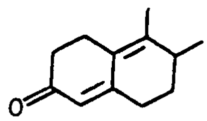

$R_1$ represents the radical:

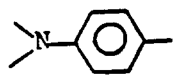

$R_2$ represents a methyl radical, $R_3$ represents a hydroxyl radical and $R_4$ represents a phenyl radical, characterized in that:
a) either a product of formula II:

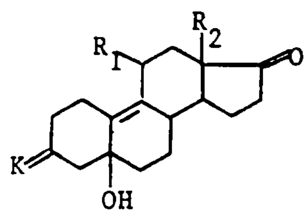

II

in which $R_1$ and $R_2$ have the meaning indicated previously and K represents a protected ketone group, is subjected

i) first, to the action of an organometallic reagent derived from an optionally substituted aryl radical that can be represented by $R_3$ or $R_4$

ii) then optionally either to a separation of the isomers obtained or to a dehydration reaction in position 16(17)

iii) then optionally, in any order, to the action of a protection, alkylation or acylation agent of the hydroxy radical that is represented by $R_3$ or $R_4$ and necessarily to the action of a dehydration agent capable of releasing the ketone function, in order to obtain the products of formula IA:

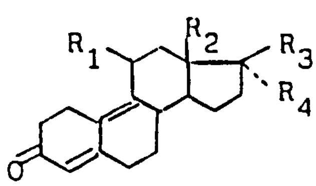

IA

and I'A

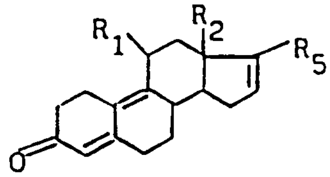

I'A

in which formulae $R_1$, $R_2$, $R_3$, $R_4$ and $R_5$ have the meaning indicated previously and which products of formula IA or I'A, if desired, are treated either by an oxidation reagent, in order to

obtain respectively a product of formula IB:

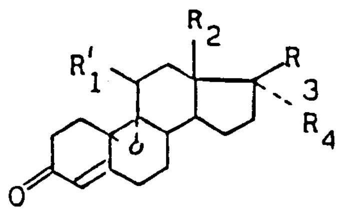

IB

and I'B:

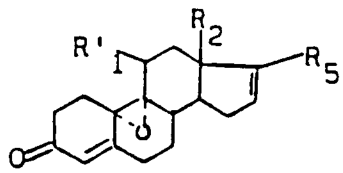

I'B

in which formulae IB and I'B $R_2$, $R_3$, $R_4$ and $R_5$ have the meaning indicated previously and $R'_1$ has the meaning indicated previously for $R_1$, it being understood however that $R'_1$ contains an oxidized nitrogen atom if $R_1$ contains a nitrogen atom, in which products of formula IB and I'B the $R'_1$ radical contains an oxidized nitrogen atom which if desired is treated by a reducing agent in order to obtain a product of formula IB or I'B in which $R'_1$ contains a non-oxidized nitrogen atom, or the products of formulae IA and I'A are treated by an aromatization agent then optionally by an alkylation or acylation reagent in order to obtain respectively the products of formula IC:

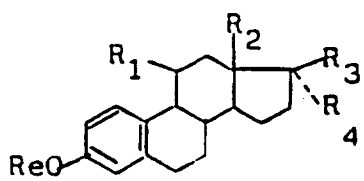

IC

and I'C

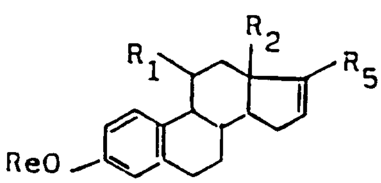

I'C

in which formulae IC and I'C Re, $R_1$, $R_2$, $R_3$, $R_4$ and $R_5$ have the meaning indicated previously,

b) or a product of formula III:

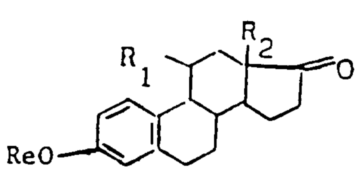

III

in which $R_1$ and $R_2$ have the meaning indicated previously, is subjected to the action of an organometallic reagent derived from an optionally substituted aryl radical that can be represented by $R_3$ or $R_4$ then optionally either in any order to a separation of the isomers obtained and to the optional action of a protection, alkylation or acylation agent of the hydroxy radical that is represented by $R_3$ or $R_4$ or to a dehydration reaction in position 16(17) and that if desired the products of formulae IA, I'A, IB, I'B, IC and I'C are subjected to the action of a base or an acid in order to obtain the corresponding salts.

**2.** Process according to claim 1, characterized in that a compound of formula (II) or (III) is used at the start, in which $R_1$ represents an optionally substituted phenyl radical.

**3.** Process according to claim 1 or 2, characterized in that products of general formula (I) are prepared in which A and B represent the group:

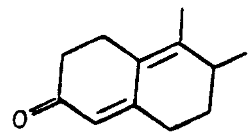

**4.** Process according to any one of claims 1 to 3, characterized in that products of general formula (I) are prepared in which $R_3$ represents a hydroxyl or methoxy radical and $R_4$ represents an optionally substituted phenyl radical.

**5.** Process according to any one of claims 1 to 4, characterized in that any one of the products are prepared of which the names follow:
- 11beta,17alpha-bis/4-(dimethylamino) phenyl/17beta-hydroxy estra-4, 9-dien-3-one,
- 11beta-/4-(dimethylamino) phenyl/ 17beta-hydroxy 17alpha-(3-methoxy phenyl) estra-4,9-dien-3-one.

**Claims for the following Contracting State : GR**

**1.** Preparation process for the products of general formula I:

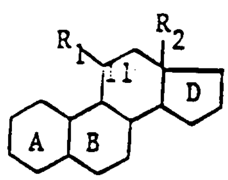

in which $R_1$ represents
- a linear or branched alkyl radical having 1 to 12 carbon atoms, or a vinyl, isopropenyl, allyl, 2-methylallyl or isobutenyl radical, all these radicals being optionally substituted by:
  . thiomethyl, thioethyl radicals,
  . halogen atoms,
  . the dimethylamino radical,
- a cycloalkyl or cycloalkenyl radical,
- a phenyl, benzyl radical or a heterocyclic aryl radical chosen from the following radicals: thienyl, furyl, isothienyl, isofuryl, thiazolyl, isothiazolyl, oxazolyl, isoxazolyl, thiadiazolyl, pyridinyl or piperidinyl, these radicals themselves being optionally substituted by one or more radicals chosen from the following radicals:
  . alkyl having 1 to 4 carbon atoms
  . alkoxy having 1 to 4 carbon atoms
  . vinyloxy or allyloxy
  . halogen atoms

. trifluoromethyl,
. alkylthio having 1 to 4 carbon atoms optionally oxidized in the form of the sulphoxide or the sulphone
. acyl such as acetyl, propionyl, butyryl or benzoyl
. an amino radical optionally substituted by one or two alkyl radicals having 1 to 8 carbon atoms or incorporated in a heterocycle optionally containing another heteroatom chosen from the group formed by oxygen, nitrogen and sulphur
. an optionally substituted amino alkyl radical such as amino methyl or aminoethyl, dimethylamino methyl or dimethylamino ethyl
. an optionally substituted amino alkyloxy radical such as dimethylamino ethyloxy
. the trimethylsilyl radical
. an optionally acylated hydroxy radical such as acetoxy or a radical of formula:

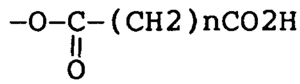

in which n = 2 to 5
. a free or esterified carboxy radical
. a cyano radical
. an aryl radical itself optionally substituted by alkyl, alkoxy, alkylthio, amino alkyl or dialkyl amino radicals as defined above,

it being understood that when the atom immediately adjacent to the carbon in position 11 is a carbon atom, $R_2$ represents a methyl or ethyl radical, rings A and B having one of the following structures:

a) either A and B represent the group:

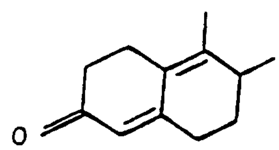

b) or A and B represent the group:

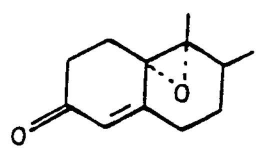

c) or A and B represent the group:

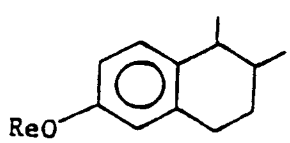

in which Re represents a hydrogen atom, an optionally substituted alkyl radical having 1 to 6 carbon atoms or an acyl radical,

ring D having one of the following structures:

a) either D represents the group:

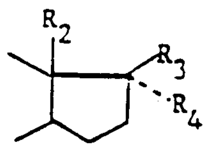

in which one of the substituents $R_3$ or $R_4$ represents an optionally protected or acylated hydroxyl radical or an alkoxyl radical and the other substituent $R_3$ or $R_4$ represents an optionally substituted aryl radical,
b) or D represents the group:

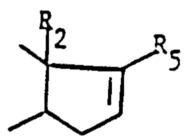

in which $R_5$ represents an optionally substituted aryl radical, as well as the addition salts of the products of formula I with the acids and bases and with the exception of the product of formula I in which rings A and B represent the group:

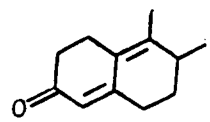

$R_1$ represents the radical:

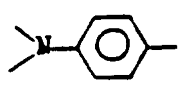

$R_2$ represents a methyl radical, $R_3$ represents a hydroxyl radical and $R_4$ represents a phenyl radical, characterized in that:
a) either a product of formula II:

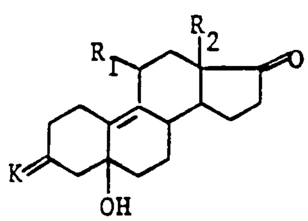

II

in which $R_1$ and $R_2$ have the meaning indicated previously and K represents a protected ketone group, is subjected

i) first, to the action of an organometallic reagent derived from an optionally substituted aryl radical that can be represented by $R_3$ or $R_4$
ii) then optionally either to a separation of the isomers obtained or to a dehydration reaction in position 16(17)

iii) then optionally, in any order, to the action of a protection, alkylation or acylation agent of the hydroxy radical that is represented by $R_3$ or $R_4$ and necessarily to the action of a dehydration agent capable of releasing the ketone function, in order to obtain the products of formula IA:

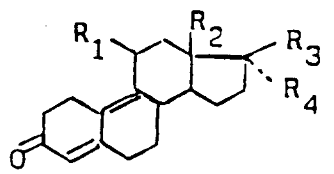

IA

and I'A

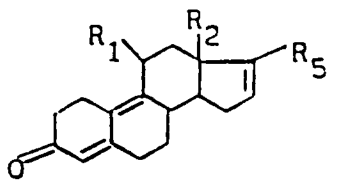

I'A

in which formulae $R_1$, $R_2$, $R_3$, $R_4$ and $R_5$ have the meaning indicated previously and which products of formula IA or I'A, if desired, are treated <u>either</u> by an oxidation reagent, in order to obtain respectively a product of formula IB:

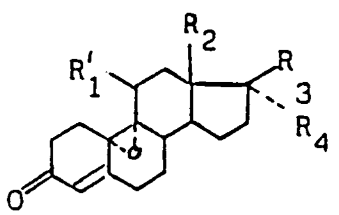

IB

and I'B:

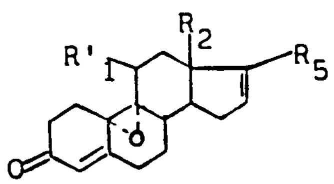

I'B

in which formulae IB and I'B $R_2$, $R_3$, $R_4$ and $R_5$ have the meaning indicated previously and $R'_1$ has the meaning indicated previously for $R_1$, it being understood however that $R'_1$ contains an oxidized nitrogen atom if $R_1$ contains a nitrogen atom, in which products of formula IB and I'B the $R'_1$ radical contains an oxidized nitrogen atom which if desired is treated by a reducing agent in order to obtain a product of formula IB or I'B in which $R'_1$ contains a non-oxidized nitrogen atom, <u>or</u> the products of formulae IA and I'A are treated by an aromatization agent then optionally by an alkylation or acylation reagent in order to obtain respectively the products of formula IC:

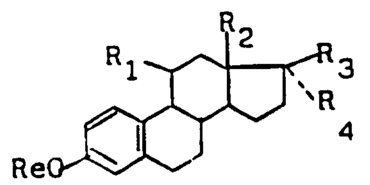

IC

and I'C

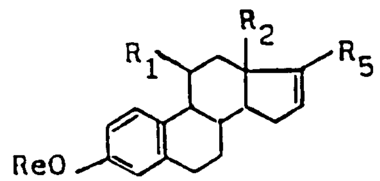

I'C

in which formulae IC and I'C Re, $R_1$, $R_2$, $R_3$, $R_4$ and $R_5$ have the meaning indicated previously,

b) or a product of formula III:

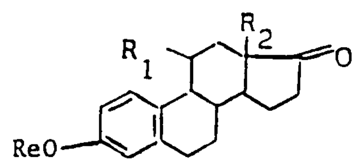

III

in which $R_1$ and $R_2$ have the meaning indicated previously, is subjected to the action of an organometallic reagent derived from an optionally substituted aryl radical that can be represented by $R_3$ or $R_4$ then optionally either in any order to a separation of the isomers obtained and to the optional action of a protection, alkylation or acylation agent of the hydroxy radical that is represented by $R_3$ or $R_4$ or to a dehydration reaction in position 16(17) and that if desired the products of formulae IA, I'A, IB, I'B, IC and I'C are subjected to the action of a base or an acid in order to obtain the corresponding salts.

**2.** Process according to claim 1, characterized in that a compound of formula (II) or (III) is used at the start, in which $R_1$ represents an optionally substituted phenyl radical.

**3.** Process according to claim 1 or 2, characterized in that products of general formula (I) are prepared in which A and B represent the group:

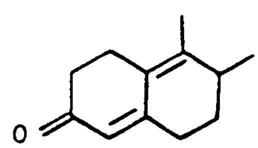

**4.** Process according to any one of claims 1 to 3, characterized in that products of general formula (I) are prepared in which $R_3$ represents a hydroxyl or methoxy radical and $R_4$ represents an optionally substituted phenyl radical.

**5.** Process according to any one of claims 1 to 4, characterized in that any one of the products are prepared of which the names follow:

- 11beta,17alpha-bis/4-(dimethylamino) phenyl/17beta-hydroxy estra-4, 9-dien-3-one,
- 11beta-/4-(dimethylamino) phenyl/ 17beta-hydroxy 17alpha-(3-methoxy phenyl) estra-4,9-dien-3-one.

**6.** Preparation process for pharmaceutical compositions characterized in that at least one of the products of general formula (I), as defined in claim 1, or at least one of their pharmaceutically acceptable addition salt with acids and bases, is used as active ingredient in a form intended for this use.

**7.** Preparation process for pharmaceutical compositions characterized in that at least one of the products of general formula (I), as defined in any one of claims 2 to 4, or at least one of their pharmaceutically acceptable addition salts with acids and bases, is used as active ingredient in a form intended for this use.

**8.** Preparation process for pharmaceutical compositions characterized in that at least one of the products of general formula (I), as defined in claim 1, of which the names follow:
- 11beta,17alpha-bis/4(dimethylamino) phenyl/17beta-hydroxy estra-4, 9-dien-3-one,
- 11beta-/4-(dimethylamino) phenyl/ 17beta-hydroxy 17alpha-(3-methoxy phenyl) estra-4,9-dien-3-one,

is used as active ingredient in a form intended for this use.

**Patentansprüche**
**Patentansprüche für folgende Vertragsstaaten : AT, BE, CH, DE, GB, IT, LI, LU, NL, SE**

**1.** Produkte der allgemeinen Formel (I)

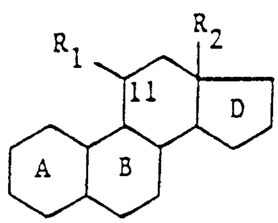

(I)

worin $R_1$ bedeutet
- einen linearen oder verzweigten Alkylrest mit 1 bis 12 Kohlenstoffatomen oder einen Vinyl-, Isopropenyl-, Allyl-2-Methylallyl- oder Isobutenylrest, wobei alle diese Reste gegebenenfalls substituiert sind durch:
  . Thiomethyl-, Thioethylreste
  . Halogenatome
  . dem Dimethylaminorest
- einen Cycloalkyl- oder Cycloalkenylrest
- einen Phenyl-, Benzylrest oder einen heterocyclischen Arylrest, ausgewählt unter den Thienyl-, Furyl-, Isothienyl, Isofuryl-, Thiazolyl-, Isothiazolyl-, Oxazolyl-, Isoxazolyl-, Thiadiazolyl-, Pyridinyl- oder Piperidinylresten, wobei diese Reste ihrerseits gegebenenfalls durch einen oder mehrere Reste substituiert sind, ausgewählt unter den Resten:
  . Alkyl mit 1 bis 4 Kohlenstoffatomen
  . Alkoxy mit 1 bis 4 Kohlenstoffatomen
  . Vinyloxy oder Allyloxy
  . den Halogenatomen
  . Trifluormethyl
  . Alkylthio mit 1 bis 4 Kohlenstoffatomen, gegebenenfalls in Form des Sulfoxids oder des Sulfons oxidiert
  . Acyl, wie Acetyl, Propionyl, Butyryl oder Benzoyl
  . einem Aminorest, gegebenenfalls substituiert durch ein oder zwei Alkylreste mit 1 bis 8 Kohlenstoffatomen, oder eingebaut in einen Heterocyclus, der gegebenenfalls ein weiteres Heteroatom, ausgewählt unter Sauerstoff, Stickstoff und Schwefel, aufweist

- . einem gegebenenfalls substituierten Aminoalkylrest, wie Aminomethyl oder Aminoethyl, Dimethylaminomethyl oder Dimethylaminoethyl
- . einem gegebenenfalls substituierten Aminoalkoxyrest, wie Dimethylaininoethyioxy
- . dem Trimethylsilylrest
- . einem gegebenenfalls acylierten Hydroxyrest, wie Acetoxy, oder einem Rest der Formel

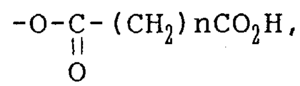

worin n = 2 bis 5

- . einem freien oder veresterten Carboxyrest
- . einer Cyanogruppe
- . einem Arylrest, der seinerseits gegebenenfalls durch Alkyl-, Alkoxy-, Alkylthio-, Aminoalkyl- oder Dialkylaminoreste, wie vorstehend definiert, substituiert sein kann

mit der Maßgabe, daß das dem Kohlenstoffatom in 11-Stellung unmittelbar benachbarte Atom ein Kohlenstoffatom ist, $R_2$ einen Methyl- oder Ethylrest bedeutet, wobei die Ringe A und B eine der folgenden Strukturen besitzen:

a) Entweder bedeuten A und B die Gruppe

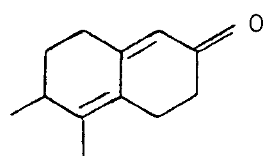

b) oder A und B bedeuten die Gruppe

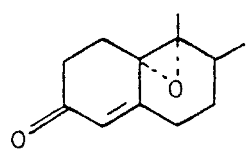

c) oder A und B bedeuten die Gruppe

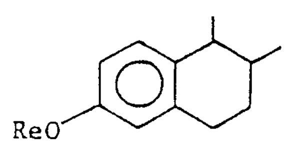

worin Re für ein Wasserstoffatom, einen gegebenenfalls substituierten Alkylrest mit 1 bis 6 Kohlenstoffatomen oder einen Acylrest steht,

wobei der Ring D eine der folgenden Strukturen aufweist:

a) Entweder bedeutet D die Gruppe

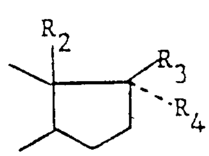

worin einer der Substituenten $R_3$ oder $R_4$ für eine gegebenenfalls geschützte oder acylierte Hydroxygruppe oder einen Alkoxyrest steht, und der andere der Substituenten $R_3$ oder $R_4$ einen

gegebenenfalls substituierten Arylrest bedeutet,
b) oder D die Gruppe

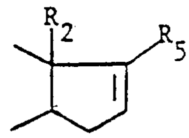

wiedergibt,
worin $R_5$ einen gegebenenfalls substituierten Arylrest bedeutet, sowie die Additionssalze der Produkte der Formel (I) mit Säuren und Basen und mit Ausnahme des Produkts der Formel (I), worin die Ringe A und B die Gruppe

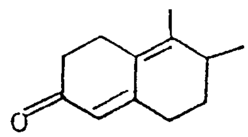

bedeuten,
$R_1$ den Rest

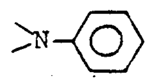

wiedergibt,
$R_2$ einen Methylrest bedeutet, $R_3$ eine Hydroxylgruppe bedeutet und $R_4$ für einen Phenylrest steht.

**2.** Produkte der allgemeinen Formel (I), wie in Anspruch 1 definiert, worin $R_1$ einen gegebenenfalls substituierten Arylrest bedeutet.

**3.** Produkte der allgemeinen Formel (I), wie in Anspruch 1 oder 2 definiert, worin A und B die Gruppe

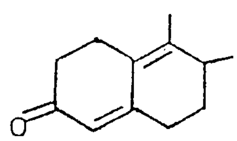

bedeuten.

**4.** Produkte der allgemeinen Formel (I), wie in einem der Ansprüche 1 bis 3 definiert, worin $R_3$ einen Kydroxyl- oder Methoxyrest bedeutet und $R_4$ für einen gegebenenfalls substituierten Arylrest steht.

**5.** Produkte der allgemeinen Formel (I), wie in einem der Ansprüche 1 bis 4 definiert, mit den folgenden Bezeichnungen:
- 11$\beta$,17$\alpha$-Bis-(dimethylamino)-phenyl-17$\beta$-hydroxy-östra-4,9-dien-3-on,
- 11$\beta$-4-(Dimethylamino)-phenyl-17$\beta$-hydroxy-17$\alpha$-(3-methoxyphenyl) -östra-4, 9-dien-3-on.

**6.** Verfahren zur Herstellung der Produkte der allgemeinen Formel (I), wie in Anspruch 1 definiert, dadurch gekennzeichnet, daß man:

a) entweder ein Produkt der Formel (II)

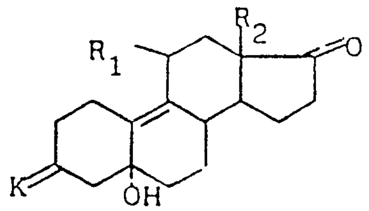

II

worin $R_1$ und $R_2$ die in Anspruch 1 angegebene Bedeutung besitzen und K eine geschützte Ketogruppe bedeutet,

i) zunächst der Einwirkung eines organometallischen Reagens, das sich von einem gegebenenfalls substituierten Arylrest ableitet, der $R_3$ oder $R_4$ bedeuten kann,

ii) dann gegebenenfalls entweder einer Trennung der erhaltenen Isomeren oder einer Dehydratation in 16(17)-Stellung,

iii) hiernach gegebenenfalls in beliebiger Reihenfolge der Einwirkung eines Mittels für den Schutz, die Alkylierung oder die Acylierung der Hydroxygruppe, die $R_3$ oder $R_4$ bedeutet, und zwangsweise der Einwirkung eines Dehydratationsmittels, das in der Lage ist, die Ketofunktion freizusetzen, unterzieht, um zu den Produkten der Formel (IA)

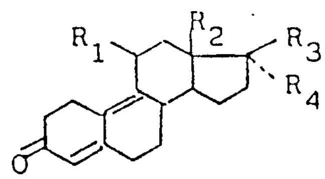

IA

und (I'A)

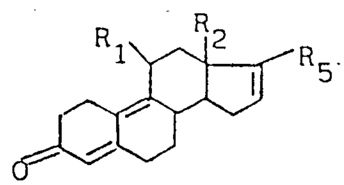

I'A

zu gelangen,

worin die Reste $R_1$, $R_2$, $R_3$, $R_4$ und $R_5$ die in Anspruch 1 angegebene Bedeutung besitzen, und die Produkte der Formel (IA) oder (I'A) gewünschtenfalls entweder mit einem Oxidationsreagens behandelt, um ein Produkt der Formel (IB)

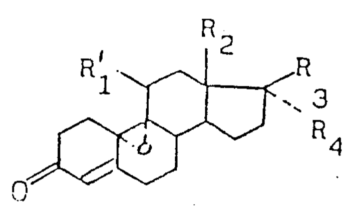

IB

bzw. (I'B)

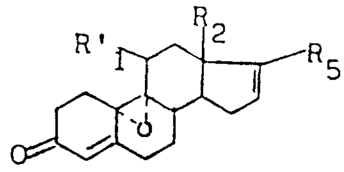

I'B

zu erhalten,
wobei in den Formeln (IB) und (I'B) $R_2$, $R_3$, $R_4$ und $R_5$ die in Anspruch 1 angegebene Bedeutung besitzen und $R'_1$ die in Anspruch 1 für $R_1$ angegebene Bedeutung besitzt, wobei es sich jedoch versteht, daß $R'_1$ ein oxidiertes Stickstoffatom aufweist, wenn $R_1$ ein Stickstoffatom enthält, die Produkte der Formel (IB) und (I'B), worin der Rest $R'_1$ ein oxidiertes Stickstoffatom enthält, gewünschtenfalls mit einem Reduktionsmittel behandelt, um zu einem Produkt der Formel (IB) bzw. (I'B) zu gelangen, worin $R'_1$ ein nicht-oxidiertes Stickstoffatom aufweist,
oder man die Produkte der Formeln (IA) und (I'A) mit einem Aromatisierungsmittel, hiernach gegebenenfalls mit einem Alkylierungs- oder Acylierungsreagens behandelt, um die Produkte der Formel (IC)

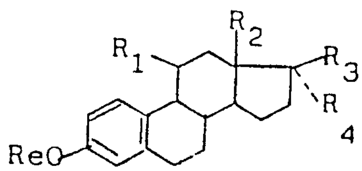

IC

bzw. (I'C)

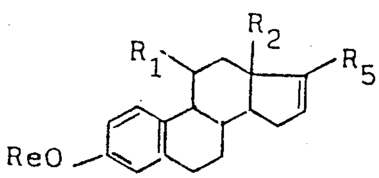

I'C

zu erhalten,
wobei in den Formeln (IC) und (I'C) Re, $R_1$, $R_2$, $R_3$, $R_4$ und $R_5$ die in Anspruch 1 angegebene Bedeutung besitzen,
b) oder ein Produkt der Formel (III)

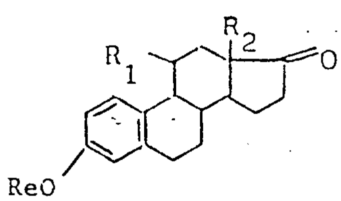

III

worin $R_1$ und $R_2$ die in Anspruch 1 angegebene Bedeutung besitzen, der Einwirkung eines organometallischen Reagens, welches sich von einem gegebenenfalls substituierten Arylrest ableitet, den $R_3$ oder $R_4$ bedeuten kann, danach gegebenenfalls entweder in beliebiger Reihenfolge einer Trennung der erhaltenen Isomeren und der etwaigen Einwirkung eines Mittels zum Schutz, zur Alkylierung oder zur Acylierung des Hydroxyrestes, den $R_3$ oder $R_4$ wiedergibt, oder einer Dehydratationsreaktion in 16(17)-Stellung unterzieht, und gewünschtenfalls die Produkte der Formeln (IA), (I'A), (IB) (I'B), (IC) und (I'C) der Einwirkung einer Base oder einer Säure unterwirft, um die entsprechenden Salze zu erhalten.

**7.** Als Arzneimittel die Produkte der allgemeinen Formel (I), wie in Anspruch 1 definiert.

**8.** Als Arzneimittel die Produkte der allgemeinen Formel (I), wie in einem der Ansprüche 2 bis 5 definiert.

**9.** Pharmazeutische Zusammensetzungen , enthaltend als Wirkstoff zumindest ein Arzneimittel, wie in einem der Ansprüche 7 oder 8 definiert.

**10.** Als neue industrielle Produkte die Produkte mit den folgenden Bezeichnungen:
Cyclisches 3,3-(1,2-Ethandiyl)-acetal von 5α-Hydroxy-11β-(4-methoxyphenyl)-östr-9-en-3,17-dion, -
Cyclisches 3,3-(1,2-Ethandiyl)-acetal von 5α-Hydroxy-11β-4-(methylthio)-phenyl-östr-9-en-3, 17-dion,
11β-4- (Dimethylamino)-phenyl-östra-4,9-dien-3, 17-dion,
11β-4-(Dimethylamino)-phenyl-3-hydroxy-östra-1,3,5(10)-trien-17-on.

**Patentansprüche für folgenden Vertragsstaat : ES**

**1.** Verfahren zur Herstellung der Produkte der allgemeinen Formel (I)

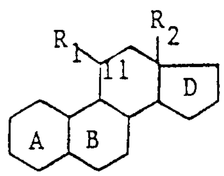

worin $R_1$ bedeutet
- einen linearen oder verzweigten Alkylrest mit 1 bis 12 Kohlenstoffatomen oder einen Vinyl-, Isopropenyl- Allyl-, 2-Methylallyl- oder Isobutenylrest, wobei alle diese Reste gegebenenfalls substituiert sind durch:
  . die Thiomethyl-, Thioethylreste
  . die Halogenatome
  . den Dimethylaminorest
- die Cycloalkyl- oder Cycloalkenylreste
- die Phenyl-, Benzylreste oder einen heterocyclischen Arylrest, ausgewählt unter den Thienyl-, Furyl-, Isothienyl-, Isofuryl-, Thiazolyl-, Isothiazolyl-, Oxazolyl-, Isoxazolyl-, Thiadiazolyl-, Pyridinyl- oder Piperidinylresten, wobei diese Reste gegebenenfalls ihrerseits durch einen oder mehrere Reste substituiert sind, ausgewählt unter den Resten:
  . Alkyl mit 1 bis 4 Kohlenstoffatomen
  . Alkoxy mit 1 bis 4 Kohlenstoffatomen
  . Vinyloxy oder Allyloxy
  . den Halogenatomen
  . Trifluormethyl
  . Alkylthio mit 1 bis 4 Kohlenstoffatomen, gegebenenfalls oxidiert in Form des Sulfoxids oder Sulfons
  . Acyl, wie Acetyl, Propionyl, Butyryl oder Benzoyl
  . einem Aminorest, gegebenenfalls substituiert durch einen oder zwei Alkylreste mit 1 bis 8 Kohlenstoffatomen, oder eingebaut in einen Heterocyclus, der gegebenenfalls ein weiteres Heteroatom, ausgewählt unter Sauerstoff, Stickstoff und Schwefel, aufweist
  . einem gegebenenfalls substituierten Aminoalkylrest, wie Aminomethyl oder Aminoethyl, Dimethylaminomethyl oder Dimethylaminoethyl
  . einem gegebenenfalls substituierten Aminoalkyloxyrest, wie Dimethylaminoethyloxy
  . dem Trimethylsilylrest
  . einem gegebenenfalls acylierten Hydroxyrest, wie Acetoxy oder einem Rest der Formel

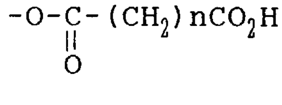

worin n = 2 bis 5

. einem freien oder veresterten Carboxyrest

. einem Cyanorest

. einem Arylrest, der seinerseits gegebenenfalls substituiert ist durch Alkyl-, Alkoxy-, Alkylthio-, Aminoalkyl-, Dialkylaminoreste, wie vorstehend definiert,

mit der Maßgabe, daß das unmittelbar dem Kohlenstoff in 11-Stellung benachbarte Atom ein Kohlenstoffatom ist, $R_2$ einen Methyl- oder Ethylrest bedeutet, die Ringe A und B eine der folgenden Strukturen aufweisen:

a) Entweder bedeuten A und B die Gruppe

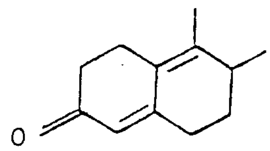

b) oder A und B bedeuten die Gruppe

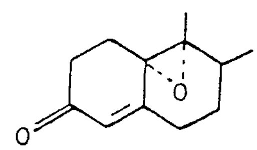

c) oder A und B bedeuten die Gruppe

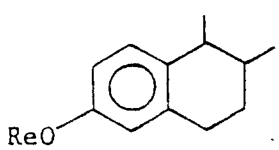

worin Re ein Wasserstoffatom, einen Alkylrest mit 1 bis 6 Kohlenstoffatomen, welcher gegebenenfalls substituiert ist, oder einen Acylrest bedeutet,

wobei der Ring D eine der folgenden Strukturen aufweist:

a) Entweder bedeutet D die Gruppe

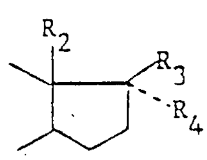

worin einer der Substituenten $R_3$ oder $R_4$ einen gegebenenfalls geschützten oder acylierten Hydroxylrest oder einen Alkoxyrest bedeutet, und der andere der Substituenten $R_3$ oder $R_4$ einen gegebenenfalls substituierten Arylrest bedeutet,

b) oder D bedeutet die Gruppe

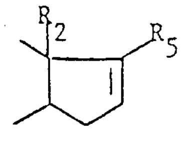

worin $R_5$ einen gegebenenfalls substituierten Arylrest darstellt, sowie von den Additionssalzen der Produkte der Formel (I) mit Säuren und Basen und mit Ausnahme des Produkts der Formel (I), worin die Ringe A und B die Gruppe

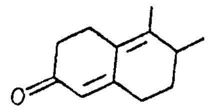

darstellen,
$R_1$ den Rest

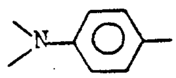

bedeutet,
$R_2$ einen Methylrest darstellt, $R_3$ eine Hydroxygruppe bedeutet und $R_4$ einen Phenylrest bedeutet, dadurch gekennzeichnet, daß man
a) entweder ein Produkt der Formel (II)

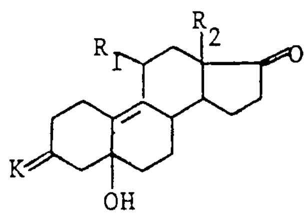

worin $R_1$ und $R_2$ die vorstehend angegebene Bedeutung besitzen und K eine geschützte Ketogruppe bedeutet,

i) zunächst der Einwirkung eines organometallischen Reagens, welches sich von einem gegebenenfalls substituierten Arylrest ableitet, den $R_3$ oder $R_4$ bedeuten kann,

ii) danach gegebenenfalls entweder einer Trennung der erhaltenen Isomeren oder einer Dehydratation in 16(17)-Stellung,

iii) anschließend gegebenenfalls in beliebiger Reihenfolge der Einwirkung eines Mittels für den Schutz, die Alkylierung oder die Acylierung des Hydroxyrestes, den $R_3$ oder $R_4$ bedeutet, und zwangsweise der Einwirkung eines Dehydratationsmittels, das in der Lage ist, die Ketofunktion freizusetzen, unterzieht, um zu den Produkten der Formel (IA)

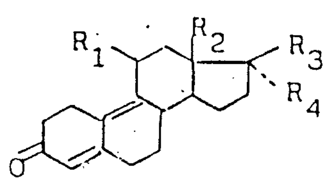

IA

und (I'A)

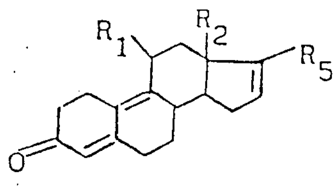

I'A

zu gelangen,
worin $R_1$, $R_2$, $R_3$, $R_4$ und $R_5$ die vorstehend angegebene Bedeutung besitzen, und die Produkte der Formeln (IA) oder (I'A) gewünschtenfalls entweder mit einem Oxidationsreagens behandelt, um zu einem Produkt der Formel (IB)

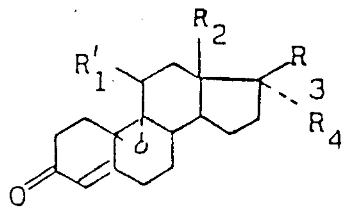

IB

bzw. (I'B)

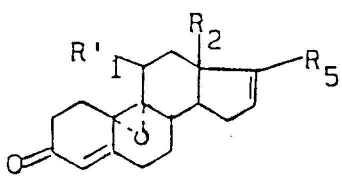

I'B

zu gelangen,
wobei in den Formeln (IB) und (I'B) $R_2$, $R_3$, $R_4$ und $R_5$ die vorstehend angegebene Bedeutung besitzen und $R'_1$ die vorstehend für $R_1$ angegebene Bedeutung besitzt, mit der Maßgabe, daß jedoch $R'_1$ ein oxidiertes Stickstoffatom bedeutet, wenn $R_1$ ein Stickstoffatom aufweist, die Produkte der Formeln (IB) und (I'B), worin der Rest $R'_1$ ein oxidiertes Stickstoffatom aufweist, gewünschtenfalls mit einem Reduktionsmittel behandelt, um zu einem Produkt der Formel (IB) oder (I'B) zu gelangen, worin $R'_1$ ein nicht-oxidiertes Stickstoffatom aufweist,
oder die Produkte der Formeln (IA) und (I'A) mit einem Aromatisierungsmittel, hiernach gegebenenfalls mit einem Alkylierungs- oder Acylierungsmittel behandelt, um die Produkte der Formel (IC)

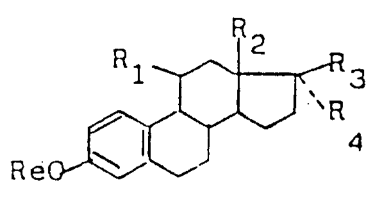

IC

bzw. (I'C)

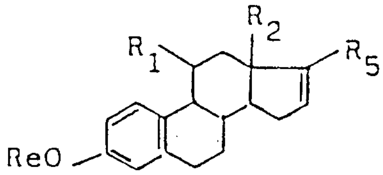

I'C

zu erhalten,
wobei in den Formeln (IC) und (I'C) Re, $R_1$, $R_2$, $R_3$, $R_4$ und $R_5$ die vorstehend angegebene Bedeutung besitzen,

b) oder ein Produkt der Formel (III)

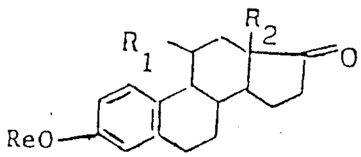

III

worin $R_1$ und $R_2$ die vorstehend angegebene Bedeutung besitzen, der Einwirkung eines organometallischen Reagens, das sich von einem gegebenenfalls substituierten Arylrest ableitet, welchen $R_3$ oder $R_4$ bedeuten kann, hiernach gegebenenfalls entweder in beliebiger Reihenfolge einer Trennung der erhaltenen Isomeren und der etwaigen Einwirkung eines Mittels für den Schutz, die Alkylierung oder die Acylierung des Hydroxyrestes, den $R_3$ oder $R_4$ darstellt, unterzieht, oder einer Dehydratationsreaktion in 16(17)-Stellung unterwirft, und gewünschtenfalls die Produkte der Formeln (IA), (I'A), (IB), (I'B), (IC) und (I'C) der Einwirkung einer Base oder einer Säure unterzieht, um die entsprechenden Salze zu erhalten.

**2.** Verfahren gemäß Anspruch 1, dadurch gekennzeichnet, daß man von einer Verbindung der Formel (II) oder (III) ausgeht, worin $R_1$ einen gegebenenfalls substituierten Phenyl-rest darstellt.

**3.** Verfahren gemäß Anspruch 1 oder 2, dadurch gekennzeichnet, daß man die Produkte der allgemeinen Formel (I) herstellt, worin A und B die Gruppe

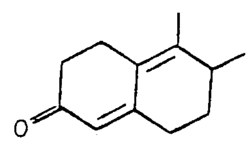

bedeuten.

**4.** Verfahren gemäß einem der Ansprüche 1 bis 3, dadurch gekennzeichnet, daß man die Produkte der allgemeinen Formel (I) herstellt, worin $R_3$ einen Hydroxyl- oder Methoxyrest bedeutet und $R_4$ einen gegebenenfalls substituierten Phenylrest darstellt.

**5.** Verfahren gemäß einem der Ansprüche 1 bis 4, dadurch gekennzeichnet, daß man eines der folgenden Produkte mit den folgenden Bezeichnungen herstellt:

- 11β,17α-Bis-4-(dimethylamino)-phenyl-17β-hydroxy-östra-4,9-dien-3-on,
- 11β-4-(Dimethylamino)-phenyl-17β-hydroxy-17α-(3-methoxyphenyl) -östra-4, 9-dien-3-on.

**Patentansprüche für folgenden Vertragsstaat : GR**

1. Verfahren zur Herstellung der Produkte der allgemeinen Formel (I)

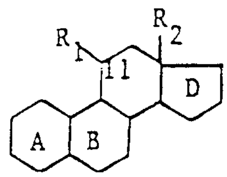

worin $R_1$ bedeutet

- einen linearen oder verzweigten Alkylrest mit 1 bis 12 Kohlenstoffatomen oder einen Vinyl-, Isopropenyl- Allyl-, 2-Methylallyl- oder Isobutenylrest, wobei alle diese Reste gegebenenfalls substituiert sind durch:
  . die Thiomethyl-, Thioethylreste
  . die Halogenatome
  . den Dimethylaminorest
- die Cycloalkyl- oder Cycloalkenylreste
- die Phenyl-, Benzylreste oder einen heterocyclischen Arylrest, ausgewählt unter den Thienyl-, Furyl-, Isothienyl-, Isofuryl-, Thiazolyl-, Isothiazolyl-, Oxazolyl-, Isoxazolyl-, Thiadiazolyl-, Pyridinyl- oder Piperidinylresten, wobei diese Reste gegebenenfalls ihrerseits durch einen oder mehrere Reste substituiert sind, ausgewählt unter den Resten:
  . Alkyl mit 1 bis 4 Kohlenstoffatomen
  . Alkoxy mit 1 bis 4 Kohlenstoffatomen
  . Vinyloxy oder Allyloxy
  . den Halogenatomen
  . Trifluormethyl
  . Alkylthio mit 1 bis 4 Kohlenstoffatomen, gegebenenfalls oxidiert in Form des Sulfoxids oder Sulfons
  . Acyl, wie Acetyl, Propionyl, Butyryl oder Benzoyl
  . einem Aminorest, gegebenenfalls substituiert durch einen oder zwei Alkylreste mit 1 bis 8 Kohlenstoffatomen, oder eingebaut in einen Heterocyclus, der gegebenenfalls ein weiteres Heteroatom, ausgewählt unter Sauerstoff, Stickstoff und Schwefel, aufweist
  . einem gegebenenfalls substituierten Aminoalkylrest, wie Aminomethyl oder Aminoethyl, Dimethylaminomethyl oder Dimethylaminoethyl
  . einem gegebenenfalls substituierten Aminoalkyloxyrest, wie Dimethylaminoethyloxy
  . dem Trimethylsilylrest
  . einem gegebenenfalls acylierten Hydroxyrest, wie Acetoxy oder einem Rest der Formel

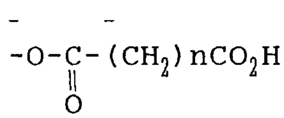

  worin n = 2 bis 5
  . einem freien oder veresterten Carboxyrest
  . einem Cyanorest
  . einem Arylrest, der seinerseits gegebenenfalls substituiert ist durch Alkyl-, Alkoxy-, Alkylthio-, Aminoalkyl-, Dialkylaminoreste, wie vorstehend definiert,

mit der Maßgabe, daß das unmittelbar dem Kohlenstoff in 11-Stellung benachbarte Atom ein Kohlenstoffatom ist, $R_2$ einen Methyl- oder Ethylrest bedeutet, die Ringe A und B eine der folgenden Strukturen aufweisen:

a) Entweder bedeuten A und B die Gruppe

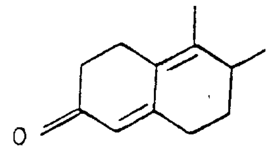

b) oder A und B bedeuten die Gruppe

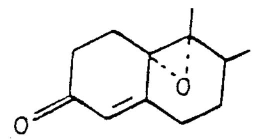

c) oder A und B bedeuten die Gruppe

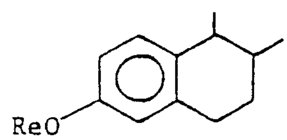

worin Re ein Wasserstoffatom, einen Alkylrest mit 1 bis 6 Kohlenstoffatomen, welcher gegebenenfalls substituiert ist, oder einen Acylrest bedeutet,
wobei der Ring D eine der folgenden Strukturen aufweist:
a) Entweder bedeutet D die Gruppe

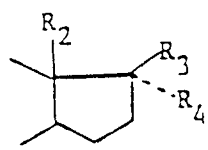

worin einer der Substituenten $R_3$ oder $R_4$ einen gegebenenfalls geschützten oder acylierten Hydroxylrest oder einen Alkoxyrest bedeutet, und der andere der Substituenten $R_3$ oder $R_4$ einen gegebenenfalls substituierten Arylrest bedeutet,
b) oder D bedeutet die Gruppe

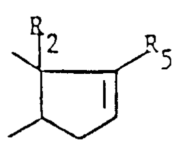

worin $R_5$ einen gegebenenfalls substituierten Arylrest darstellt, sowie von den Additionssalzen der Produkte der Formel (I) mit Säuren und Basen und mit Ausnahme des Produkts der Formel (I), worin die Ringe A und B die Gruppe

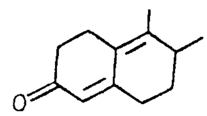

darstellen,
$R_1$ den Rest

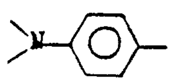

bedeutet,
$R_2$ einen Methylrest darstellt, $R_3$ eine Hydroxygruppe bedeutet und $R_4$ einen Phenylrest bedeutet, dadurch gekennzeichnet, daß man
a) entweder ein Produkt der Formel (II)

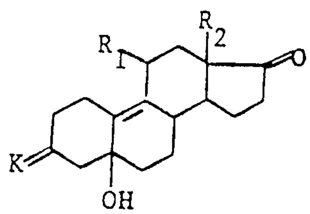

II

worin $R_1$ und $R_2$ die vorstehend angegebene Bedeutung besitzen und K eine geschützte Ketogruppe bedeutet,

i) zunächst der Einwirkung eines organometallischen Reagens, welches sich von einem gegebenenfalls substituierten Arylrest ableitet, den $R_3$ oder $R_4$ bedeuten kann,
ii) danach gegebenenfalls entweder einer Trennung der erhaltenen Isomeren oder einer Dehydratation in 16(17)-Stellung,
iii) anschließend gegebenenfalls in beliebiger Reihenfolge der Einwirkung eines Mittels für den Schutz, die Alkylierung oder die Acylierung des Hydroxyrestes, den $R_3$ oder $R_4$ bedeutet, und zwangsweise der Einwirkung eines Dehydratationsmittels, das in der Lage ist, die Ketofunktion freizusetzen, unterzieht, um zu den Produkten der Formel (IA)

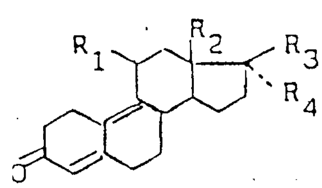

IA

und (I'A)

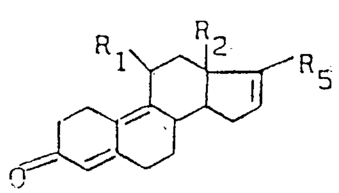

I'A

zu gelangen,
worin $R_1$, $R_2$, $R_3$, $R_4$ und $R_5$ die vorstehend angegebene Bedeutung besitzen, und die Produkte

der Formeln (IA) oder (I'A) gewünschtenfalls entweder mit einem Oxidationsreagens behandelt, um zu einem Produkt der Formel (IB)

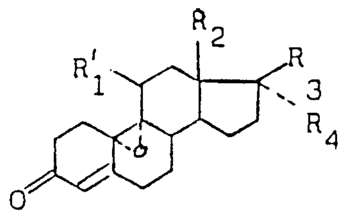

IB

bzw. (I'B)

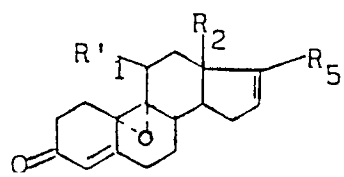

I'B

zu gelangen,
wobei in den Formeln (IB) und (I'B) $R_2$, $R_3$, $R_4$ und $R_5$ die vorstehend angegebene Bedeutung besitzen und $R'_1$ die vorstehend für $R_1$ angegebene Bedeutung besitzt, mit der Maßgabe, daß jedoch $R'_1$ ein oxidiertes Stickstoffatom bedeutet, wenn $R_1$ ein Stickstoffatom aufweist, die Produkte der Formeln (IB) und (I'B) , worin der Rest $R'_1$ ein oxidiertes Stickstoffatom aufweist, gewünschtenfalls mit einem Reduktionsmittel behandelt, um zu einem Produkt der Formel (IB) oder (I'B) zu gelangen, worin $R'_1$ ein nicht-oxidiertes Stickstoffatom aufweist,
oder die Produkte der Formeln (IA) und (I'A) mit einem Aromatisierungsmittel, hiernach gegebenenfalls mit einem Alkylierungs- oder Acylierungsmittel behandelt, um die Produkte der Formel (IC)

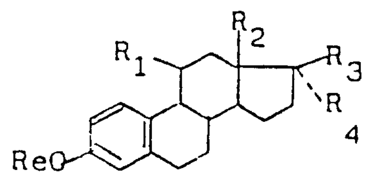

IC

bzw. (I'C)

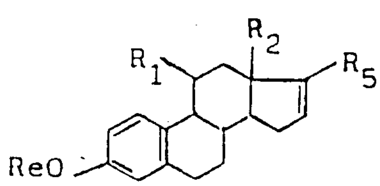

I'C

zu erhalten,
wobei in den Formeln (IC) und (I'C) Re, $R_1$, $R_2$, $R_3$, $R_4$ und $R_5$ die vorstehend angegebene Bedeutung besitzen,

b) oder ein Produkt der Formel (III)

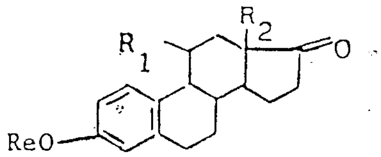

III

worin $R_1$ und $R_2$ die vorstehend angegebene Bedeutung besitzen, der Einwirkung eines organometallischen Reagens, das sich von einem gegebenenfalls substituierten Arylrest ableitet, welchen $R_3$ oder $R_4$ bedeuten kann, hiernach gegebenenfalls entweder in beliebiger Reihenfolge einer Trennung der erhaltenen Isomeren und der etwaigen Einwirkung eines Mittels für den Schutz, die Alkylierung oder die Acylierung des Hydroxyrestes, den $R_3$ oder $R_4$ darstellt, unterzieht, oder einer Dehydratationsreaktion in 16(17)-Stellung unterwirft, und gewünschtenfalls die Produkte der Formeln (IA), (I'A), (IB), (I'B), (IC) und (I'C) der Einwirkung einer Base oder einer Säure unterzieht, um die entsprechenden Salze zu erhalten.

**2.** Verfahren gemäß Anspruch 1, dadurch gekennzeichnet, daß man von einer Verbindung der Formel (II) oder (III) ausgeht, worin $R_1$ einen gegebenenfalls substituierten Phenylrest darstellt.

**3.** Verfahren gemäß Anspruch 1 oder 2, dadurch gekennzeichnet, daß man die Produkte der allgemeinen Formel (I) herstellt, worin A und B die Gruppe

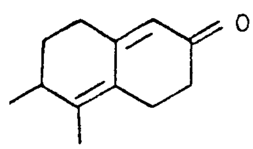

bedeuten.

**4.** Verfahren gemäß einem der Ansprüche 1 bis 3, dadurch gekennzeichnet, daß man die Produkte der allgemeinen Formel (I) herstellt, worin $R_3$ einen Hydroxyl- oder Methoxyrest bedeutet und $R_4$ einen gegebenenfalls substituierten Phenylrest darstellt.

**5.** Verfahren gemäß einem der Ansprüche 1 bis 4, dadurch gekennzeichnet, daß man eines der folgenden Produkte mit den folgenden Bezeichnungen herstellt:

- 11β,17α-Bis-4-(dimethylamino)phenyl-17β-hydroxy-östra-4,9-dien-3-on,
- 11β-4-(Dimethylamino)-phenyl-17β-hydroxy-17α-(3-methoxyphenyl)-östra-4,9-dien-3-on.

**6.** Verfahren zur Herstellung von pharmazeutischen Zusammensetzungen, dadurch gekennzeichnet, daß man als Wirkstoff zumindest eines der Produkte der allgemeinen Formel (I), wie in Anspruch 1 definiert, oder zumindest eines ihrer Additionssalze mit pharmazeutisch verträglichen Säuren und Basen in eine für diese Verwendung bestimmte Form überführt.

**7.** Verfahren zur Herstellung von pharmazeutischen Zusammensetzungen, dadurch gekennzeichnet, daß man als Wirkstoff zumindest eines der Produkte der allgemeinen Formel (I), wie in einem der Ansprüche 2 bis 4 definiert, oder zumindest eines ihrer Additionssalze mit pharmazeutisch verträglichen Säuren und Basen in eine für diese Verwendung bestimmte Form überführt.

**8.** Verfahren zur Herstellung von pharmazeutischen Zusammensetzungen, dadurch gekennzeichnet, daß man als Wirkstoff zumindest eines der Produkte der allgemeinen Formel (I), wie in Anspruch 1 definiert, mit den folgenden Bezeichnungen:

- 11β,17α-Bis-4-(dimethylamino)-phenyl-17β-hydroxy-östra-4,9-dien-3-on,
- 11β-4-(Dimethylamino)-phenyl-17β-hydroxy-17α-(3-methoxyphenyl)-östra-4, 9-dien-3-on,

in eine für diese Verwendung bestimmte Form überführt.